(19) Europäisches Patentamt
European Patent Office
Office européen des brevets

(11) EP 0 714 386 B1

(12) **FASCICULE DE BREVET EUROPEEN**

(45) Date de publication et mention de la délivrance du brevet:
**10.03.1999 Bulletin 1999/10**

(21) Numéro de dépôt: **95922359.5**

(22) Date de dépôt: **16.06.1995**

(51) Int Cl.[6]: **C07C 311/08**, C07C 229/26, C08B 37/00, A61K 31/63, A61K 47/48

(86) Numéro de dépôt international:
**PCT/BE95/00055**

(87) Numéro de publication internationale:
**WO 95/34533 (21.12.1995 Gazette 1995/54)**

(54) **SEL DE NIMESULIDE HYDROSOLUBLE ET LEURS UTILISATIONS POUR LE TRAITEMENT DES AFFECTIONS INFLAMMATOIRES**

WASSERLÖSLICHES NIMESULID-SALZ, SOWIE DESSEN VERWENDUNG ZUR BEHANDLUNG VON ENTZÜNDUNGEN

WATER-SOLUBLE NIMESULIDE SALT, AND USES THEREOF FOR THE TREATMENT OF INFLAMMATIONS

(84) Etats contractants désignés:
**AT BE CH DE DK ES FR GB GR IE IT LI LU MC NL PT SE**
Etats d'extension désignés:
**LT LV SI**

(30) Priorité: **16.06.1994 BE 9400582**

(43) Date de publication de la demande:
**05.06.1996 Bulletin 1996/23**

(73) Titulaire: **EUROPHARMACEUTICALS S.A.**
**B-1180 Bruxelles (BE)**

(72) Inventeurs:
- **PIROTTE, Bernard**
  **B-4680 Oupeye (BE)**
- **PIEL, Géraldine**
  **B-4020 Liège (BE)**
- **NEVEN, Philippe**
  **B-4460 Grâce-Hollogne (BE)**
- **DELNEUVILLE, Isabelle**
  **B-4430 Ans (BE)**
- **GECZY, Joszef**
  **B-1180 Bruxelles (BE)**

(74) Mandataire: **Schmitz, Yvon**
**Gevers & Vander Haeghen,**
**Patent Attorneys,**
**Rue de Livourne 7**
**1060 Brussels (BE)**

(56) Documents cités:
**WO-A-91/17774** **FR-A- 2 190 460**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 714 386 B1

## Description

La présente invention concerne un sel de nimésulide hydrosoluble et sa préparation, une solution aqueuse le contenant, des associations à base de nimésulide avec les cyclodextrines, ainsi que les utilisations de ces sel de nimésulide, solution aqueuse et associations à base de nimésulide.

Comme on le sait, le nimésulide, également connu sous la dénomination de N-(4-nitro-2-phénoxyphényl) méthanesulfonamide ou 4-nitro-2-phénoxyméthanesulfonanilide, est un médicament bien connu utilisé dans le traitement des maladies ou affections inflammatoires, tels que, par exemple, les affections rhumatismales, les inflammations aiguës. Il présente toutefois l'inconvénient d'être très peu soluble dans l'eau, ce qui contrecarre son utilisation dans certaines applications galéniques telles que solutés buvables et injectables, et certaines autres préparations orales, telles que comprimés ou granulés effervescents. D'autre part, il est connu que les sels de nimésulide en solution connus jusqu'à présent, en particulier le sel sodique, imposent un pH relativement alcalin dont il faut être averti lors de leur formulation sous forme injectable. De plus, le sel sodique de nimésulide, bien que soluble, présente l'inconvénient de libérer avec lui des ions sodiques qui sont souvent contre-indiqués. Il est également connu, suivant les demandes de brevet WO 91/17774 et 94/02177, de pouvoir complexer le nimésulide avec des cyclodextrines mais en fait ceux-ci ne permettent qu'une augmentation modérée de la solubilité dans l'eau du nimésulide non salifié, c'est-à-dire de l'ordre d'environ 0,05 mg/ml (valeur déterminée par diagramme de solubilité), soit 5 fois la solubilité du nimésulide (0,01 mg/ml).

La présente invention a pour but de remédier à ces inconvénients et de prévoir un sel de nimésulide fortement soluble dans l'eau, qui puisse notamment servir à la préparation de solutés buvables ou injectables de nimésulide, tout en conservant les applications pharmacologiques et/ou thérapeutiques anti-inflammatoires du nimésulide acide.

A cet effet, suivant l'invention, le sel est constitué par le produit de réaction du nimésulide et de la L-lysine.

Avantageusement, le sel contient environ 1 mole de L-lysine par mole de nimésulide.

Suivant une forme de réalisation avantageuse, on prévoit une solution aqueuse dudit sel de nimésulide en combinaison avec de la L-arginine, le rapport pondéral du sel de nimésulide à la L-arginine étant avantageusement d'environ 1/1.

L'invention se rapporte également à des associations à base de nimésulide afin d'augmenter encore sensiblement l'hydrosolubilité du nimésulide par rapport au sel de nimésulide-L-lysine.

A cet égard, on associe suivant l'invention le nimésulide et la L-lysine à au moins une cyclodextrine, sous la forme d'un mélange de ces composés, d'un mélange du sel de nimésulide-L-lysine avec la cyclodextrine, d'un complexe de nimésulide-cyclodextrine en mélange avec de la L-lysine ou encore d'un complexe de nimésulide-L-lysine-cyclodextrine.

Suivant une autre forme de réalisation avantageuse de l'invention, la cyclodextrine est choisie dans le groupe comprenant les $\alpha$-, $\beta$- et $\gamma$-cyclodextrines, leurs hydrates, leurs dérivés et leurs mélanges.

Enfin, l'invention se rapporte aussi à la préparation du sel de nimésulide susmentionné ainsi qu'à ses applications et aux applications de la solution aqueuse le contenant et des associations précitées.

Suivant un mode de réalisation, on dissout le nimésulide et la L-lysine dans du méthanol et on élimine le méthanol du mélange ainsi obtenu par des méthodes classiques, telles que concentration par évaporation, puis filtration, le méthanol utilisé pour dissoudre le nimésulide étant chauffé de préférence à une température proche de son point d'ébullition, avantageusement à une température de 54 à 64°C.

Comme on vient de le préciser, le sel de nimésulide hydrosoluble de la présente invention est constitué par le produit de la réaction du nimésulide avec l'acide aminé L-lysine sous sa forme non salifiée monohydratée. Ce sel de nimésulide et de L-lysine ou sel de nimésulide-L-lysine contient 1 mole de L-lysine par mole de nimésulide. Il est avantageux de respecter le rapport mole à mole de nimésulide et de L-lysine lors de la préparation du sel en solution méthanolique car si l'on s'en écarte tout excès de l'un ou l'autre partenaire de la réaction acide-base est une source de contamination pour le produit final. La réaction acide-base pour la préparation du sel de nimésulide-L-lysine est conforme au schéma réactionnel suivant :

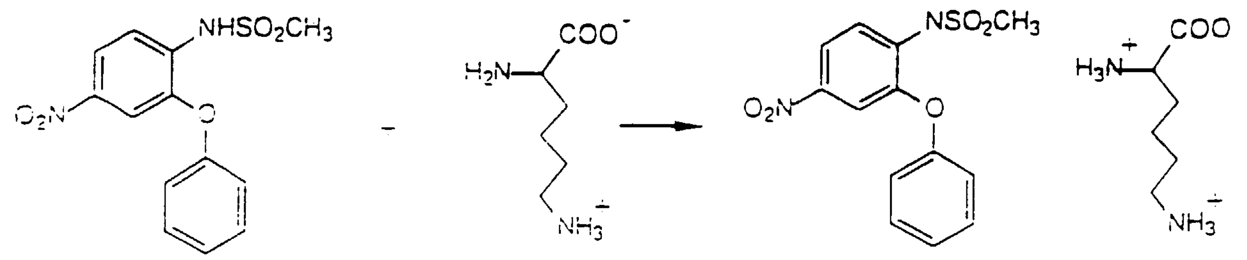

Nimésulide L-lysine Sel de nimésulide-L-lysine

Les conditions opératoires de la réaction précitée sont les suivantes. Le nimésulide est dissous à chaud dans le méthanol, de préférence à une température proche du point d'ébullition du méthanol, avantageusement à une température de 54 à 64°C. La L-lysine, quant à elle, est également dissoute dans du méthanol, de préférence également dans du méthanol chauffé à une température de 54 à 64°C mais ceci n'est pas obligatoire. En effet, on pourrait concevoir la préparation d'une solution méthanolique de L-lysine à température ambiante, qu'on verse ensuite dans la solution méthanolique chaude de nimésulide. De toute façon, le méthanol chaud permet de dissoudre à la fois le nimésulide acide et la L-lysine. Par conséquent, la réaction entre l'acide (nimésulide) et la base (L-lysine) se réalise par la mise en contact ou le mélange de deux solutions, donnant une solution du sel de nimésulide-L-lysine qui, par concentration et repos à basse température, laisse cristalliser le sel attendu. Celui-ci recueilli sur filtre peut alors être lavé au méthanol, ce qui laisse supposer que les contaminants du sel (nimésulide acide en excès non transformé ou, l'inverse, lysine en excès non transformée) seront éliminés par ce lavage au méthanol car ils sont relativement solubles dans celui-ci.

En ce qui concerne la durée de mise en contact des deux solutions méthanoliques, celle-ci est extrêmement brève puisqu'en principe, une réaction acide-base est instantanée.

Suivant l'invention, on peut également améliorer sensiblement la solubilité du sel de nimésulide-L-lysine dans l'eau en l'associant avec de la L-arginine. On obtient des résultats particulièrement satisfaisants lorsque l'on utilise des solutions aqueuses de sel de nimésulide-L-lysine dans lesquelles le rapport pondéral du sel de nimésulide à la L-arginine est de l'ordre de 1/1.

L'utilisation simultanée de la L-lysine et des cyclodextrines permet d'augmenter, suivant l'invention, la solubilité du nimésulide d'une valeur maximale supérieure à 20 mg/ml (valeur déterminée par diagramme de solubilité), soit plus de 2.000 fois la solubilité du nimésulide. Les associations à base de nimésulide de l'invention consistent en des mélanges ou des complexes de nimésulide, de L-lysine et de cyclodextrine. C'est ainsi qu'elles peuvent être sous la forme d'un mélange de nimésulide, de L-lysine et de cyclodextrine, d'un mélange de sel de nimésulide-L-lysine et de cyclodextrine, d'un complexe de nimésulide-cyclodextrine en mélange avec de la L-lysine ou encore d'un complexe de nimésulide-L-lysine-cyclodextrine.

On utilisera avantageusement comme cyclodextrines les $\alpha$-, $\beta$- et $\gamma$-cyclodextrines, leurs hydrates, leurs dérivés comme les dérivés de cyclodextrine alkylés et hydroxyalkylés et leurs mélanges. Le rapport molaire nimésulide-L-lysine-cyclodextrine varie avantageusement entre 1/1/1 et 1/2/1.

Ces associations à base de nimésulide sont préparées en solution aqueuse et sont obtenues à l'état solide par concentration sous pression réduite de la solution ou encore par toute autre technique telle que la lyophilisation, la nébulisation.

On donne ci-après des exemples de préparation du sel de nimésulide-L-lysine et de solution aqueuse utilisant le sel de nimésulide-L-lysine en combinaison avec de la L-arginine ainsi que d'associations de nimésulide-L-lysine-cyclodextrine sous forme de complexes ou de simples mélanges.

**<u>Exemple 1</u> : Préparation du sel de nimésulide-L-lysine**

On dissout du nimésulide (3,08 g; 0,01 mole) dans du méthanol chaud (100 ml) maintenu à 60°C puis on l'additionne d'une solution méthanolique (50 ml) de L-lysine "hydrate" (Janssen Chimica; 1,64 g; 0,01 mole calculée sur la lysine monohydratée). La solution orangée obtenue est éventuellement filtrée à chaud pour éliminer un léger trouble persistant. Le filtrat méthanolique limpide est concentré à l'évaporateur rotatif jusqu'à un volume de 80 ml. La suspension de sel obtenue est refroidie à une température de +4°C pendant 2 heures. Le précipité est recueilli sur filtre en verre fritté, lavé deux fois avec un petit volume de méthanol puis séché (production de ± 3 g). Le filtrat orangé additionné d'éther diéthylique (150 ml) et refroidi à une température de -30°C pendant deux jours abandonne un deuxième jet de sel moins pur.

Point de fusion du sel de nimésulide préparé en solution méthanolique : 200-204°C avec décomposition. P. F. du deuxième jet précipité à l'éther : 188-201°C avec décomposition.

| Analyse élémentaire | |
|---|---|
| analyse théorique : | N : 12,33 |
| | C : 50,21 |
| | H : 5,77 |
| | S : 7,05 |
| sel (méthanol) : | N : 12,43 |
| | C : 50,07 |
| | H : 5,88 |
| | S : 6,80 |
| sel (éther) | N : 12,65 |
| | C : 50,11 |
| | H : 6,18 |
| | S : 6,35 |

Les spectres I.R. et R.M.N. confirment que le sel de nimésulide-L-lysine est effectivement un nouveau composé aux propriétés physico-chimiques différentes de celles d'un mélange physique à parties égales de nimésulide et de L-lysine.

Spectres I.R.

- Figure 1 : spectre du nimésulide
- Figure 2 : spectre de la L-lysine anhydre
- Figure 3 : spectre du sel de nimésulide-L-lysine de l'invention
- Figure 4 : spectre d'un mélange physique de nimésulide et de L-lysine
- Figure 5 : superposition des spectres des figures 1, 2 et 3 entre 800 et 1800 $cm^{-1}$.

On observe en particulier que la bande d'absorption N-H d'allongement à 3284 $cm^{-1}$ présente sur le spectre de la figure 1 du nimésulide (concerne donc le fragment $-SO_2NH-$ impliqué dans la salification), ne se retrouve pas sur le spectre de la figure 3 du sel (puisque, en principe, ce fragment devient $-SO_2N^{(-)}$ dans la structure saline), mais s'observe sur le spectre de la figure 4 du mélange physique des partenaires.

D'autre part, le spectre de la figure 5, qui visualise la superposition des spectres des figures 1, 2 et 3 entre 800 et 1800 $cm^{-1}$, démontre que le sel (a) présente des bandes d'absorption différentes de celles du nimésulide acide (b) et de la lysine (c). En particulier, pour ne citer que celles-là, les bandes d'absorption nettes vers 900 $cm^{-1}$, vers 1150 $cm^{-1}$ et vers 1250 $cm^{-1}$ du nimésulide acide ont disparu sur le spectre du sel.

On peut donc conclure qu'un certain nombre de liaisons aux caractéristiques d'absorption de l'I.R. nouvelles se sont formées dans le sel témoignant probablement de la déprotonation de la fonction $-SO_2NH-$ du nimésulide.

Spectres R.M.N.

- Figure 6 : spectre du nimésulide acide dans le DMSO deutéré (+ HMDS) entre 0 et 10 ppm.
- Figure 7 : spectre du nimésulide acide dans le DMSO deutéré (+ HMDS) entre 5 et 15 ppm.
- Figure 8 : spectre du sel de nimésulide avec la L-lysine dans le DMSO deutéré (+ HMDS) entre 0 et 10 ppm.
- Figure 9 : spectre du sel de nimésulide avec la L-lysine dans le DMSO deutéré (+ HMDS) entre 5 et 15 ppm.

Le spectre de la figure 7 montre la présence pour le nimésulide acide d'un proton fortement déblindé (acide)

correspondant au proton de la fonction $-SO_2NH-$.

Comparé au spectre de la figure 7, le spectre de la figure 9 du sel montre la disparition vers 10 ppm du signal du proton $-SO_2NH-$.

D'autre part, comparé au spectre de la figure 6, le spectre de la figure 7 fait apparaître des signaux supplémentaires correspondant aux protons C-H de la lysine.

On observe également que la salification du nimésulide par la lysine génère une fonction $SO_2N^{(-)}$ qui, compte tenu de l'augmentation de la densité électronique liée à la charge négative, induit un blindage généralisé des protons de la partie nimésulide. En particulier, les trois protons méthyle présents sur le spectre de la figure 6 vers 3,0 ppm pour l'acide, se retrouvent déplacés vers 2,5 ppm pour le sel.

**Exemple 2 : Préparation d'une solution aqueuse de sel de nimésulide-L-lysine avec addition de L-arginine**

Pour réaliser la mise en solution de 10 mg de nimésulide par millilitre de solution aqueuse, on utilise le sel de nimésulide-L-lysine tel que préparé dans l'Exemple 1 (15 mg) additionné de 15 mg de L-arginine. Cette association du sel de nimésulide-L-lysine avec de la L-arginine permet d'augmenter sensiblement la solubilité du sel dans l'eau. On notera à cet égard que le sel de nimésulide-L-lysine dissous dans l'eau relargue en partie du nimésulide acide insoluble.

Le sel de nimésulide-L-lysine soluble dans l'eau de la présente invention exerce une activité anti-inflammatoire remarquable dans toutes les affections inflammatoires où le nimésulide acide est utilisé. C'est ainsi que le sel de l'invention peut servir à la préparation de solutés buvables ou injectables de nimésulide moyennant une formulation adéquate, par exemple avec l'adjonction de L-arginine. On notera à cet égard que les sels de nimésulide connus (sel sodique ou sels d'amine) en solution imposent un pH relativement alcalin dont il faut être averti lors de leur formulation sous forme injectable. Par contre, le sel de nimésulide L-lysine associé ou non à de la L-arginine impose en solution un pH nettement moins alcalin que les sels de métaux alcalins, ce qui constitue, comme on le sait, un avantage très important par rapport à ces derniers.

Le sel de nimésulide-L-lysine hydrosoluble de l'invention peut être administré en association avec divers excipients pharmaceutiques, tels que des diluants, des gélifiants, des agents conservateurs, des émulsionnants, des édulcorants et aromatisants et cela par voie orale, parentérale ou rectale.

Pour une administration orale, on utilisera des dragées, granulés, pastilles, tablettes, capsules, comprimés, solutions, sirops, émulsions contenant des additifs ou excipients classiques en pharmacie galénique. Ces formes galéniques peuvent libérer le principe de façon normale ou programmée dans le temps.

Pour l'administration parentérale, on utilisera tout véhicule approprié, comme, par exemple de l'eau stérile.

Pour l'administration rectale, on utilisera des suppositoires, des capsules rectales, des solutions ou des gelées.

Le composé actif peut être administré seul ou en combinaison avec d'autres produits actifs ayant une activité similaire ou différente.

L'exemple suivant met en évidence le rôle des β et γ-cyclodextrines (β-CD et γ-CD) sur la solubilité du sel de nimésulide-L-lysine.

**Exemple 3 : Diagramme de solubilité du sel de nimésulide-L-lysine en fonction de la quantité de β- et γ-CD.**

Un excès de sel nimésulide-L-lysine est mis en suspension dans 20 ml d'eau contenant des concentrations croissantes de β- ou γ-CD (0, 10, 20, 30, 40, 50, 100, 200, 300 mM). L'ensemble est agité à 20°C pendant 10 jours. Les mélanges sont ensuite filtrés au travers d'une membrane de 0,45 μm. Le nimésulide dissous est dosé par spectrophotométrie à 397 nm après dilution appropriée dans une solution NaOH 0,1N.

Le Tableau 1 ci-après résume les résultats obtenus :

Tableau 1

| Concentration en β- ou γ-CD | Nimésulide dissous en présence de β-CD | | Nimésulide dissous en présence de γ-CD | |
|---|---|---|---|---|
| (mM) | mg/ml | mM | mg/ml | mM |
| 0 | 5,42 | 17,60 | 5,42 | 17,60 |
| 10 | 6,97 | 22,60 | 8,75 | 28,40 |
| 20 | 8,60 | 27,90 | 12,00 | 38,90 |
| 30 | 10,41 | 33,80 | 15,22 | 49,40 |
| 40 | 11,77 | 38,20 | 17,68 | 57,30 |

Tableau 1 (suite)

| Concentration en β- ou γ-CD | Nimésulide dissous en présence de β-CD | | Nimésulide dissous en présence de γ-CD | |
|---|---|---|---|---|
| (mM) | mg/ml | mM | mg/ml | mM |
| 50 | 16,32 | 52,90 | 23,63 | 76,60 |
| 100 | 25,31 | 82,10 | 30,27 | 98,20 |
| 200 | 25,23 | 81,80 | 30,16 | 97,80 |

Ces résultats sont représentés à la Figure 10.

**<u>Exemple 4</u> : Préparation de l'association nimésulide-L-lysine-β-CD dans le rapport moléculaire 1/1/1.**

2,63 g de β-CD.10$H_2O$ (2 x $10^{-3}$ mole) et 0,62 g de nimésulide (2 x $10^{-3}$ mole) sont mis en suspension dans 40 ml d'eau et amenés à 85-90°C sous agitation. A la suspension est ajoutée par petites portions de la L-lysine monohydratée jusqu'à dissolution complète (0,48-0,50 g de L-lysine : 2,9-3 x $10^{-3}$ mole). La solution est concentrée à l'évaporateur rotatif jusqu'à l'obtention d'un liquide visqueux translucide (± 8 ml). Celui-ci est abandonné pendant 24 heures à température ambiante. Un précipité cristallin apparaît lentement. Le lendemain, le précipité est recueilli sur filtre, lavé avec un minimum d'eau distillée et séché. On recueille ± 1,27 g d'un produit jaune cristallin.

L'association nimésulide-L-lysine-β-CD peut être obtenue au départ du sel de nimésulide-L-lysine et de β-CD en ajoutant progressivement de la L-lysine monohydratée jusqu'à dissolution complète d'une suspension chauffée à 85-90°C. On obtient un même rapport de fractions molaires totales de β-CD, de nimésulide et de L-lysine engagées.

<u>Analyses du composé obtenu</u> :

| Association nimésulide-L-lysine-β-CD.10$H_2O$ | | Teneur théorique | Valeur mesurée |
|---|---|---|---|
| Analyse élémentaire : | %N | 3,17 | 3,57 |
| | %C | 41,39 | 42,06 |
| | %H | 6,61 | 7,15 |
| | %S | 1,81 | 1,67 |
| Teneur en eau (%)* | | 10,17 | 10,40 |
| Teneur en nimésulide (%)** | | 17,42 | 17,51 |

* mesurée par la méthode Karl Fisher

** dosée par spectrophotométrie visible à 397 nm après dilution appropriée dans du NaOH 0,1N.

Les analyses élémentaires (C, H, N, S) et les analyses de teneur en eau et en nimésulide indiquent que le composé solide obtenu correspond pratiquement à une combinaison de sel nimésulide-L-lysine et de β-CD dans un rapport moléculaire 1/1/1 et associée à 10 molécules d'eau de cristallisation. Donc, l'excès de L-lysine utilisé est éliminé lors de la filtration du solide.

**<u>Exemple 5</u> : Préparation du complexe d'inclusion nimésulide-L-lysine-β-CD par nébulisation.**

6,16 g de nimésulide (2 x $10^{-2}$ mole) sont mis en suspension dans 200 ml d'eau distillée à laquelle sont ajoutés 6,56 g de L-lysine (4 x$10^{-2}$ mole). La suspension est soumise aux ultrasons pendant 5 minutes puis chauffée à 50°C tout en maintenant une agitation violente [Ultraturrax (marque déposée)]. 26,3 g de β-CD.10$H_2O$ (2 x $10^{-2}$ mole) sont dispersés dans 200 ml d'eau distillée et chauffés à 50°C. La suspension de β-CD est ajoutée à la suspension de nimésulide et de L-lysine. L'agitation est maintenue pendant 15 minutes. On obtient une solution orangée.

Cette solution est nébulisée (atomiseur Niro Atomizer Mobile) dans les conditions suivantes :

| | |
|---|---|
| Pression | 2-3 bars |
| Température d'entrée | 150-160°C |
| Température de sortie | 60-70°C |

(suite)

| | |
|---|---|
| Débit | ± 25 ml/min |

Le rendement est de ± 80 %.

Le produit obtenu est une poudre jaune vif, sans odeur et correspond donc à un complexe ou une combinaison nimésulide-L-lysine-β-CD.$8H_2O$ (1/2/1) ou plus probablement à un complexe nimésulide-L-lysine-β-CD.$8H_2O$ (1/1/1) avec un équivalent moléculaire de L-lysine en excès.

L'analyse élémentaire donne les valeurs suivantes :

| % de | Valeur théorique* | Valeur mesurée |
|---|---|---|
| C | 42,85 | 42,84 |
| H | 6,72 | 6,64 |
| N | 4,48 | 4,34 |
| S | 1,70 | 1,38 |

* Pour une association nimésulide-L-lysine-β-CD.$8H_2O$ (1/2/1).

Le produit isolé présente les caractéristiques suivantes :

| | |
|---|---|
| Solubilité dans l'eau | 350 mg/ml (60,7 mg de nimésulide/ml) |
| Solubilité dans HCl 0,1N | 48,02 μg de nimésulide/ml |
| Solubilité à pH 6,8 | 2,37 mg de nimésulide/ml |
| pH d'une solution à 2 % | 8,85-8,95 |
| Teneur en eau | 7,6 % |
| Teneur en nimésulide | 15,67 %. |

Le spectre U.V. entre 250 et 500 nm (Figure 11) montre que tout le nimésulide est sous forme ionisée (nimésulide-L-lysine) car il n'y a pas de maximum d'absorption à 297 nm correspondant au maximum d'absorption du nimésulide acide.

<u>Preuves d'inclusion</u> :

**Analyses thermiques différentielles (ATD)** : La figure 12 représente le thermogramme d'analyse thermique différentielle (ATD) du produit obtenu par nébulisation. Le thermogramme ATD du sel de nimésulide-L-lysine (figure 13) présente un endotherme de fusion vers 208°C, la L-lysine (figure 14) présente principalement un endotherme vers 225°C. Un mélange physique de sel de nimésulide-L-lysine + L-lysine + β-CD réalisé de façon à obtenir les mêmes fractions moléculaires que les produits nébulisés (figure 15) permet de retrouver l'endotherme caractéristique du sel et de la L-lysine.

L'endotherme avant 100°C correspond à la perte en eau. On notera que le complexe (figure 12) ne présente plus l'endotherme caractéristique du sel de nimésulide-L-lysine, ce qui tend à prouver l'inclusion à l'état solide du sel de nimésulide-L-lysine dans la β-CD. Par contre, on retrouve un excès de L-lysine vers 225°C et la perte en eau avant 100°C.

**<u>Exemple 6</u> : Préparation du complexe d'inclusion nimésulide-L-lysine-γ-CD par nébulisation.**

3 g de nimésulide ($9,73 \times 10^{-3}$ mole) sont mis en suspension dans 200 ml d'eau distillée à laquelle sont ajoutés 3,2 g de L-lysine ($1,95 \times 10^{-2}$ mole). La suspension est soumise aux ultrasons pendant 5 minutes puis chauffée à 50°C tout en maintenant une agitation violente [Ultraturrax (marque déposée)]. 13,88 g de γ-CD.$7H_2O$ ($9,73 \times 10^{-3}$ mole) sont dispersés dans 200 ml d'eau distillée et chauffés à 50°C. La suspension de γ-CD est ajoutée à la suspension de nimésulide + L-lysine. L'agitation est maintenue pendant 15 minutes. On obtient une solution orangée. Cette solution est nébulisée (atomiseur Niro Atomizer Mobile) dans les conditions suivantes :

| | |
|---|---|
| Pression | 2-3 bars |
| Température d'entrée | 150-160°C |
| Température de sortie | 60-70°C |

(suite)

| | |
|---|---|
| Débit | ± 25 ml/min. |

Le rendement est de ± 80 %.

Le produit obtenu est une poudre jaune vif, sans odeur et présente les caractéristiques suivantes :

| | |
|---|---|
| Solubilité dans l'eau | 300 mg/ml (47,67 mg de nimésulide/ml) |
| Solubilité dans HCl 0,1N | 20,28 µg de nimésulide/ml |
| Solubilité à pH 6,8 | 1,71 mg de nimésulide/ml |
| pH d'une solution à 2 % | 8,98 |
| Teneur en eau | 5,98 % |
| Teneur en nimésulide | 14,94 %. |

<u>Preuves d'inclusion</u> :

**Analyses thermiques différentielles (ATD)** : La figure 16 montre le thermogramme d'analyse thermique différentielle (ATD) du complexe et la figure 17 le thermogramme d'analyse thermique différentielle (ATD) d'un mélange nimésulide-L-lysine + L-lysine + γ-CD. Pour le complexe, on retrouve la perte en eau en dessous de 100°C et l'excès de L-lysine à ± 227°C mais pas d'endotherme vers 208°C correspondant au sel nimésulide-L-lysine. L'inclusion est mise en évidence par cette technique à l'état solide.

L'ATD montre que le nimésulide sous forme de sel nimésulide-L-lysine est pratiquement totalement inclus dans la β- et γ-CD sous la forme d'un complexe d'inclusion, et que la combinaison solide isolée par nébulisation est l'association de ce complexe d'inclusion avec un excès de L-lysine.

**<u>Exemple 7</u> : Preuve d'inclusion dans la β-CD en solution par RMN$^1$H.**

Afin de prouver l'existence possible d'une inclusion en solution aqueuse, on a réalisé une étude par RMN$^1$H 80 Mhz et 400 Mhz dans $D_2O$. La mise en solution du sel de nimésulide L-lysine et de l'association nimésulide-L-lysine-β-CD (1/1/1) a été effectuée en présence d'un excès de L-lysine (deux équivalents de L-lysine pour un équivalent de nimésulide) afin d'éviter tout risque de libération d'une petite quantité de nimésulide acide très peu soluble dans l'eau ainsi que pour se trouver dans les mêmes proportions stoechiométriques que les complexes préparés par nébulisation. Les spectres ont été pris entre 0 et 10 ppm.

Figure 18 : spectre RMN$^1$H 80 Mhz du sel de nimésulide L-lysine dans $D_2O$ entre 0 et 10 ppm, en présence d'un excès de L-lysine.

Figure 19 : spectre RMN$^1$H 80 Mhz de la β-CD dans $D_2O$ entre 0 et 10 ppm.

Figure 20 : spectre RMN$^1$H 80 Mhz du complexe nimésulide-L-lysine-β-CD dans $D_2O$ entre 0 et 10 ppm, en présence d'un excès de L-lysine.

Le spectre de la figure 18 montre la présence entre 7 et 8,5 ppm des protons aromatiques du nimésulide, vers 4,7 ppm du HOD, vers 3 ppm la présence de protons blindés du nimésulide (trois protons méthyle à proximité du $SO_2N$) et du groupe méthylène ($CH_2$-N) de la lysine. Vers 1,5-2 ppm, on retrouve d'autres protons de la L-lysine.

Le spectre de la figure 19 montre vers 4,7 ppm du HOD et vers 3-4 ppm, des protons de la cyclodextrine.

Le spectre de la figure 20 (complexe) permet de retrouver les différents groupes de protons caractéristiques, avec quelques déplacements.

Les figures 21 et 22 montrent la partie entre 3 et 4,5 ppm caractéristique des protons de la β-CD, de respectivement, la β-CD et le complexe. La comparaison de ces spectres montre clairement le déplacement des pics dû à l'inclusion du nimésulide dans la β-CD.

Les figures 23 et 24 montrent la partie entre 6 et 9 ppm caractéristique des protons des noyaux aromatiques du nimésulide. La comparaison de ces spectres montre le déplacement des pics caractéristiques des protons aromatiques du nimésulide.

Les analyses par RMN$^1$H 400 Mhz confirment ces premiers résultats.

Figure 25 : spectre RMN$^1$H 400 Mhz de la β-CD dans $D_2O$.

Figure 26 : spectre RMN$^1$H 400 Mhz du mélange nimésulide-L-lysine-β-CD 1/2/1 dans $D_2O$, partie caractéristique de la β-CD.

La figure 25 permet de distinguer les différents protons de la β-CD. On remarque sur la figure 25 que les protons 3-H et 5-H sont les plus affectés. Or, ce type de comportement est typique de l'inclusion de molécules aromatiques dans les cyclodextrines. En effet, 3-H et 5-H sont les protons localisés à l'intérieur de la cavité de la cyclodextrine.

Figure 27 : spectre RMN$^1$H 400 Mhz du sel de nimésulide-L-lysine + excès de L-lysine, partie caractéristique de la partie aromatique.

Figure 28 : spectre RMN$^1$H 400 Mhz du mélange nimésulide-L-lysine-β-CD 1/2/1.

Ici aussi, on constate des déplacements de protons caractéristiques des parties aromatiques du nimésulide. Le cycle n° 2 présente un groupe de protons du type AA'BB'C qui pourrait être attribué aux groupes de pics a et b. Puisque ces groupes de protons conservent la même symétrie en présence de β-CD, ceci veut dire que le cycle 2 conserve sa libre rotation et n'est donc pas inclus. Ce serait donc le cycle n° 1 qui serait inclus dans la β-CD, en solution.

**Exemple 8 : Comparaison des solubilités à différents pH**

Le Tableau 2 suivant compare la solubilité en fonction du pH du nimésulide, du sel de nimésulide-L-lysine, de la combinaison nimésulide-L-lysine-β-CD (1/2/1) obtenue par nébulisation, de la combinaison nimésulide-L-lysine-γ-CD (1/2/1) obtenue par nébulisation ainsi que la solubilité de mélanges de sel de nimésulide-L-lysine avec un équivalent de L-lysine supplémentaire et de la β- ou γ-CD afin de respecter les mêmes proportions que les combinaisons précitées.

**Tableau 2**

**Solubilité exprimée en µg de nimésulide par ml.**

| pH | 1,5 | 3,0 | 4,0 | 5,0 | 6,0 | 6,8 |
|---|---|---|---|---|---|---|
| nimésulide | 4,8 | 4,6 | 4,6 | 4,9 | 7,0 | 14,8 |
| nimésulide-L-lysine | 8,5 | 5,1 | 4,3 | 4,6 | 9,9 | 41,7 |
| Complexes : | | | | | | |
| nimésulide-L-lysine-ß-CD 1/2/1 nébulisé | 48,0 | 37,9 | 31,1 | 46,1 | 504,6 | 2373,0 |
| nimésulide-L-lysine-γ-CD 1/2/1 nébulisé | 20,3 | 11,3 | 11,1 | 12,5 | 62,4 | 1711,2 |
| Mélanges physiques : | | | | | | |
| nimésulide-L-lysine + L-lysine + ß-CD | 31,9 | 21,2 | 24,8 | 45,1 | 470,9 | 2391,6 |
| nimésulide-L-lysine + L-lysine + γ-CD | 10,2 | 8,4 | 8,8 | 8,5 | 68,3 | 1766,2 |

On constate que complexes et mélanges physiques donnent des résultats comparables. La complexation a donc lieu in situ, en solution. C'est pourquoi on utilise également la L-lysine comme simple excipient.

**Exemple 9 : Etude de l'influence de la L-lysine sur l'augmentation de la solubilité dans l'eau d'une combinaison solide issue de l'atomisation d'une solution aqueuse ammoniacale de nimésulide et de β-CD.**

20 g de nimésulide sont dispersés dans 350 ml d'eau. 85,4 g de β-CD sont dispersés dans 800 ml d'eau distillée. Les deux suspensions sont réunies et on ajoute 20 ml de solution ammoniacale à 16,7 %. On obtient une solution qui est nébulisée dans les conditions suivantes :

| | |
|---|---|
| Débit | ± 14 ml/min. |
| Température d'entrée | 140-150°C |
| Température de sortie | 80°C. |

Le produit isolé présente les caractéristiques suivantes :
Poudre jaune vif à odeur d'ammoniaque.

| | |
|---|---|
| Teneur en eau | 5,06 % |
| Teneur en nimésulide | 20,03 % |
| Teneur en $NH_3/NH_4^+$ | 0,48 % |

2 g du produit isolé (1,3 x $10^{-3}$ mole de nimésulide) sont suspendus dans 40 ml d'eau et maintenus à température de 25°C durant l'addition progressive de L-lysine monohydratée. La dissolution complète du solide est atteinte lorsque la quantité de L-lysine monohydratée est égale à 0,30 g ± 0,02 g (1,8 x $10^{-3}$ mole).

La même manipulation effectuée à 80°C donne un résultat comparable (remise en solution totale) avec l'ajout de 0,21 ± 0,02 g de L-lysine monohydratée (1,3 x $10^{-3}$ mole). Les deux solutions jaunes conservées à température ambiante durant plusieurs jours n'abandonnent aucun précipité.

Dans cette expérience, il est démontré à nouveau que l'utilisation simultanée de L-lysine et de cyclodextrine permet d'améliorer considérablement la solubilité du nimésulide.

On donne ci-après quelques exemples non limitatifs de compositions pharmaceutiques à base de nimésulide suivant la présente invention.

**Exemple 10 : Comprimés ou gélules à 50 ou 100 mg de nimésulide.**

| | 50 mg de nimésulide | 100 mg de nimésulide |
|---|---|---|
| Complexe de nimésulide-L-lysine-β-CD (1/2/1) | 266,5 mg | 533,0 mg |
| Carbonate calcique | 54,0 mg | 108,0 mg |
| Explotab (marque déposée) (superdésintégrant) | 18,0 mg | 36,0 mg |
| Avicel PH 200 (marque déposée) (cellulose microcristalline) | 18,0 mg | 36,0 mg |
| TOTAL : | 356,5 mg | 713 mg |

**Exemple 11 : Comprimés ou gélules à 50 ou 100 mg de nimésulide:**

| | 50 mg de nimésulide | 100 mg de nimésulide |
|---|---|---|
| Nimésulide-L-lysine | 73,7 mg | 147,4 mg |
| β-cyclodextrine | 184,2 mg | 368,5 mg |
| L-lysine | 23,7 mg | 47,4 mg |
| Carbonate calcique | 57,1 mg | 114,2 mg |
| Explotab (marque déposée) | 19,0 mg | 38,0 mg |

(suite)

| | 50 mg de nimésulide | 100 mg de nimésulide |
|---|---|---|
| Avicel PH 200 (marque déposée) | 19,0 mg | 38,0 mg |
| TOTAL : | 376,7 mg | 753,4 mg |

**<u>Exemple 12</u> : Comprimés ou gélules à 50 ou 100 mg de nimésulide.**

| | 50 mg de nimésulide | 100 mg de nimésulide |
|---|---|---|
| Nimésulide | 50,0 mg | 100 mg |
| β-cyclodextrine | 184,1 mg | 368,2 mg |
| L-lysine | 47,4 mg | 94,8 mg |
| Carbonate calcique | 57,1 mg | 114,2 mg |
| Explotab (marque déposée) | 19,0 mg | 38,0 mg |
| Avicel PH 200 (marque déposée) | 19,0 mg | 38,0 mg |
| TOTAL : | 376,6 mg | 753,2 mg |

**<u>Exemple 13</u> : Comprimés ou gélules à 50 ou 100 mg de nimésulide.**

| | 50 mg de nimésulide | 100 mg de nimésulide |
|---|---|---|
| Nimésulide β-CD | 249,6 mg | 499,2 mg |
| L-lysine | 47,4 mg | 94,8 mg |
| Carbonate calcique | 57,1 mg | 114,2 mg |
| Explotab (marque déposée) | 19,0 mg | 38,0 mg |
| Avicel PH 200 (marque déposée) | 19,0 mg | 38,0 mg |
| TOTAL : | 392,1 mg | 784,2 mg |

Une granulation à sec des constituants des Exemples 10, 11, 12 et 13 donne un granulé qui peut être soit comprimé après addition de 1 % de stéarate de magnésium soit mis en gélules soit encore mis en sachets :

| Granulé | équivalent à 50 mg de nimésulide | équivalent à 100 mg de nimésulide |
|---|---|---|
| Sorbitol | 2500 mg | 4000 mg |
| Essence de citron | 15 mg | 30 mg |
| Saccharine Na | 5 mg | 5 mg |

Pour les Exemples 11, 12 et 13, le complexe est formé in situ.

**<u>Exemple 15</u> : Formulation liquide orale**

| | |
|---|---|
| Complexe nimésulide-L-lysine-β-CD (1/2/1) | 5,330 g |
| Hydroxypropylcellulose | 0,600 g |
| Méthylparaben | 0,210 g |
| Propylparaben | 0,090 g |

(suite)

| | |
|---|---|
| Saccharine sodique | 0,100 g |
| Eau | quantité suffisante pour faire 300 ml |

L'hydroxypropylcellulose est dissoute dans environ 80 ml d'eau tiède contenant le complexe dissous. Les autres additifs sont ajoutés de façon à obtenir une solution homogène. Chaque cuillère à soupe (15 ml) contient 50 mg de nimésulide.

**<u>Exemple 16</u> : Formulation d'une composition injectable contenant 5 mg/ml de nimésulide.**

| | |
|---|---|
| Complexe nimésulide-L-lysine-γ-CD (1/2/1) | 334,67 mg |
| NaCl quantité suffisante pour faire une solution isotonique | (± 70 mg) |
| Eau pour injection | 10 ml |

La γ-CD utilisée pour la préparation est une γ-CD de qualité apyrogène. La solution est introduite dans un flacon approprié après filtration stérilisante.

Les différentes formulations s'appliquent donc soit aux complexes directement, soit aux mélanges de façon à former le complexe soluble in situ.

D'autres formulations sont également possibles tels que suppositoires, pommades.

**Revendications**

1. Sel de nimésulide hydrosoluble, caractérisé en ce qu'il est constitué par le produit de réaction du nimésulide et de la L-lysine.

2. Sel suivant la revendication 1, caractérisé en ce qu'il contient 1 mole de L-lysine par mole de nimésulide.

3. Solution aqueuse, de sel de nimésulide, caractérisée en ce qu'elle comprend le sel de nimésulide suivant l'une ou l'autre des revendications 1 et 2 et de la L-arginine.

4. Solution suivant la revendication 3, caractérisée en ce que le rapport pondéral du sel de nimésulide à la L-arginine est d'environ 1/1.

5. Association à base de nimésulide, caractérisée en ce qu'elle comprend le mélange de nimésulide, de L-lysine et d'au moins une cyclodextrine.

6. Association à base de nimésulide, caractérisée en ce qu'elle comprend le sel de nimésulide-L-lysine suivant l'une ou l'autre des revendications 1 et 2 en mélange avec au moins une cyclodextrine.

7. Association à base de nimésulide, caractérisée en ce qu'elle comprend le complexe de nimésulide avec au moins une cyclodextrine en mélange avec de la L-lysine.

8. Association à base de nimésulide, caractérisée en ce qu'elle comprend un complexe de nimésulide-L-lysine-cyclodextrine.

9. Association suivant l'une quelconque des revendications 5 à 8, caractérisée en ce que la cyclodextrine est choisie dans le groupe comprenant les α-, β- et γ-cyclodextrines, leurs hydrates, leurs dérivés et leurs mélanges.

10. Association suivant la revendication 9, caractérisée en ce que les dérivés de cyclodextrine sont choisis parmi les dérivés de cyclodextrine alkylés et hydroxyalkylés.

11. Association suivant l'une quelconque des revendications 5 à 10, caractérisée en ce que le rapport molaire nimésulide-L-lysine-cyclodextrine est de 1/1/1 à 1/2/1.

**12.** Procédé de préparation du sel de nimésulide hydrosoluble suivant l'une ou l'autre des revendications 1 et 2, caractérisé en ce qu'on dissout le nimésulide et la L-lysine dans du méthanol et l'on sépare le méthanol du mélange ainsi obtenu par des méthodes de séparation telles que par filtration et/ou concentration par évaporation.

**13.** Procédé suivant la revendication 12, caractérisé en ce qu'on prépare deux solutions méthanoliques distinctes contenant respectivement le nimésulide et la L-lysine, qu'on met en contact ensuite pour obtenir le mélange précité.

**14.** Procédé suivant l'une ou l'autre des revendications 12 et 13, caractérisé en ce qu'on utilise pour dissoudre au moins le nimésulide, du méthanol chauffé à une température proche du point d'ébullition de ce dernier.

**15.** Procédé suivant la revendication 14, caractérisé en ce qu'on chauffe le méthanol à une température de 54 à 64°C.

**16.** Composition pharmaceutique pour le traitement des affections inflammatoires, comprenant une quantité efficace du sel de nimésulide suivant l'une ou l'autre des revendications 1 et 2 et/ou préparé suivant l'une quelconque des revendications 12 à 15 ou d'une solution aqueuse de sel de nimésulide suivant l'une ou l'autre des revendications 3 et 4 ou d'une association à base de nimésulide suivant l'une quelconque des revendications 5 à 11, associée à au moins un excipient approprié et à d'éventuels autres agents thérapeutiques.

**17.** Composition pharmaceutique suivant la revendication 16, caractérisée en ce que dans le cas d'une association à base de nimésulide, elle contient comme excipient, au moins de la L-lysine.

**18.** Utilisation du sel de nimésulide suivant l'une ou l'autre des revendications 1 et 2 et/ou préparé suivant l'une quelconque des revendications 12 à 15 ou de la solution aqueuse de sel de nimésulide suivant l'une ou l'autre des revendications 3 et 4 ou d'une association à base de nimésulide suivant l'une quelconque des revendications 5 à 11, dans la préparation d'un médicament pour le traitement des affections inflammatoires.

**Patentansprüche**

**1.** Wasserlösliches Nimesulidsalz, dadurch gekennzeichnet, daß es aus dem Reaktionsprodukt zwischen Nimesulid und L-Lysin gebildet ist.

**2.** Salz gemäß Anspruch 1, dadurch gekennzeichnet, daß es 1 mol L-Lysin je mol Nimesulid enthält.

**3.** Wäßrige Nimesulidsalzlösung, dadurch gekennzeichnet, daß sie das Nimesulidsalz nach einem der Ansprüche 1 und 2 und L-Arginin umfaßt.

**4.** Lösung gemäß Anspruch 3, dadurch gekennzeichnet, daß das Gewichtsverhältnis zwischen dem Nimesulidsalz und L-Arginin ungefähr 1:1 ist.

**5.** Assoziat auf der Basis von Nimesulid, dadurch gekennzeichnet, daß es die Mischung aus Nimesulid, L-Lysin und mindestens ein Cyclodextrin umfaßt.

**6.** Assoziat auf der Basis von Nimesulid, dadurch gekennzeichnet, daß es das Nimesulid-L-Lysin-Salz gemäß einem der Ansprüche 1 und 2 in einer Mischung mit wenigstens einem Cyclodextrin umfaßt.

**7.** Assoziat auf der Basis von Nimesulid, dadurch gekennzeichnet, daß es den Nimesulidkomplex mit wenigstens einem Cyclodextrin in einer Mischung mit L-Lysin umfaßt.

**8.** Assoziat auf der Basis von Nimesulid, dadurch gekennzeichnet, daß es einen Komplex aus Nimesulid-L-Lysin-Cyclodextrin umfaßt.

**9.** Assoziat gemäß einem der Ansprüche 5 bis 8, dadurch gekennzeichnet, daß das Cyclodextrin ausgewählt ist aus der Gruppe umfassend die $\alpha$-, $\beta$- und $\gamma$-Cyclodextrine, ihre Hydrate, ihre Derivate und ihre Mischungen.

**10.** Assoziat gemäß Anspruch 9, dadurch gekennzeichnet, daß die Derivate von Cyclodextrin aus den alkylierten und hydroxyalkylierten Cyclodextrinderivaten ausgewählt sind.

11. Assoziat gemäß einem der Ansprüche 5 bis 10, dadurch gekennzeichnet, daß das molare Verhältnis von Nimesulid zu L-Lysin zu Cyclodextrin 1:1:1 bis 1:2:1 ist.

12. Verfahren zur Herstellung des wasserlöslichen Nimesulidsalzes nach einem der Ansprüche 1 und 2, dadurch gekennzeichnet, daß man Nimesulid und L-Lysin in Methanol löst und daß man das Methanol von der derart erhaltenen Mischung durch Trennverfahren wie Filtration und/oder Konzentrierung mittels Verdampfen trennt.

13. Verfahren nach Anspruch 12, dadurch gekennzeichnet, daß man zwei verschiedene methanolische Lösungen herstellt, die jeweils Nimesulid und L-Lysin enthalten, die man anschließend vereinigt, um die oben erwähnte Mischung zu erhalten.

14. Verfahren nach einem der Ansprüche 12 und 13, dadurch gekennzeichnet, daß man bis nahe an den Verdampfungspunkt erhitztes Methanol verwendet, um mindestens das Nimesulid in Lösung zu bringen.

15. Verfahren nach Anspruch 14, dadurch gekennzeichnet, daß man das Methanol auf eine Temperatur von 54 bis 64 °C erhitzt.

16. Pharmazeutische Zusammensetzung zur Behandlung von Entzündungen, umfassend eine wirksame Menge des Nimesulidsalzes nach einem der Ansprüche 1 und 2 und/oder hergestellt nach einem der Ansprüche 12 bis 15 oder hergestellt aus einer wäßrigen Lösung des Nimesulidsalzes nach einem der Ansprüche 3 und 4 oder aus einem Assoziat auf der Basis von Nimesulid nach einem der Ansprüche 5 bis 11, in Verbindung mit mindestens einem geeigneten Grundstoff und eventuellen anderen therapeutischen Mitteln.

17. Pharmazeutische Zusammensetzung gemäß Anspruch 16, dadurch gekennzeichnet, daß sie im Falle eines Assoziats auf der Basis von Nimesulid als Grundstoff wenigstens L-Lysin enthält.

18. Verwendung des Nimesulidsalzes nach einem der Ansprüche 1 und 2 und/oder hergestellt nach einem der Ansprüche 12 bis 15 oder aus einer wäßrigen Lösung des Nimesulidsalzes nach einem der Ansprüche 3 und 4 oder eines Assoziats auf der Basis von Nimesulid nach einem der Ansprüche 5 bis 11 zur Herstellung eines Medikamentes zur Behandlung von Entzündungen.

**Claims**

1. Water-soluble nimesulide salt characterized in that it consists of the product of reaction of nimesulide and of L-lysine.

2. Salt according to Claim 1, characterized in that it contains 1 mole of L-lysine per mole of nimesulide.

3. Aqueous solution of nimesulide salt, characterized in that it includes the nimesulide salt according to either of Claims 1 and 2 and L-arginine.

4. Solution according to Claim 3, characterized in that the weight ratio of the nimesulide salt to the L-arginine is approximately 1/1.

5. Nimesulide-based combination characterized in that it includes the mixture of nimesulide, of L-lysine and of at least one cyclodextrin.

6. Nimesulide-based combination characterized in that it includes the nimesulide-L-lysine salt according to either of Claims 1 and 2 as a mixture with at least one cyclodextrin.

7. Nimesulide-based combination characterized in that it includes the complex of nimesulide with at least one cyclodextrin as a mixture with L-lysine.

8. Nimesulide-based combination characterized in that it includes a nimesulide-L-lysine-cyclodextrin complex.

9. Combination according to any one of Claims 5 to 8, characterized in that the cyclodextrin is chosen from the group including $\alpha$-, $\beta$- and $\gamma$-cyclodextrins, their hydrates, their derivatives and their mixtures.

**10.** Combination according to Claim 9, characterized in that the cyclodextrin derivatives are chosen from alkylated and hydroxyalkylated cyclodextrin derivatives.

**11.** Combination according to any one of Claims 5 to 10, characterized in that the nimesulide-L-lysine-cyclodextrin molar ratio is from 1/1/1 to 1/2/1.

**12.** Process for the preparation of the water-soluble nimesulide salt according to either of Claims 1 and 2, characterized in that nimesulide and L-lysine are dissolved in methanol and the methanol is separated from the mixture thus obtained by methods of separation such as by filtration and/or concentration by evaporation.

**13.** Process according to Claim 12, characterized in that two separate methanolic solutions are prepared containing nimesulide and L-lysine respectively, which are subsequently placed in contact to obtain the above-mentioned mixture.

**14.** Process according to either of Claims 12 and 13, characterized in that methanol heated to a temperature close to the boiling point of the latter is employed for dissolving at least the nimesulide.

**15.** Process according to Claim 14, characterized in that the methanol is heated to a temperature of 54 to 64°C.

**16.** Pharmaceutical composition for the treatment of inflammatory disorders, including an effective quantity of the nimesulide salt according to either of Claims 1 and 2 and/or prepared according to any one of Claims 12 to 15 or of an aqueous solution of nimesulide salt according to either of Claims 3 and 4 or of a nimesulide-based combination according to any one of Claims 5 to 11, used in combination with at least one appropriate excipient and with optional other therapeutic agents.

**17.** Pharmaceutical composition according to Claim 16, characterized in that in the case of a nimesulide-based combination it contains at least L-lysine as excipient.

**18.** Use of the nimesulide salt according to either of Claims 1 and 2 and/or prepared according to any one of Claims 12 to 15 or of the aqueous solution of nimesulide salt according to either of Claims 3 and 4 or of a nimesulide-based combination according to any one of Claims 5 to 11, in the preparation of a medication for the treatment of inflammatory disorders.

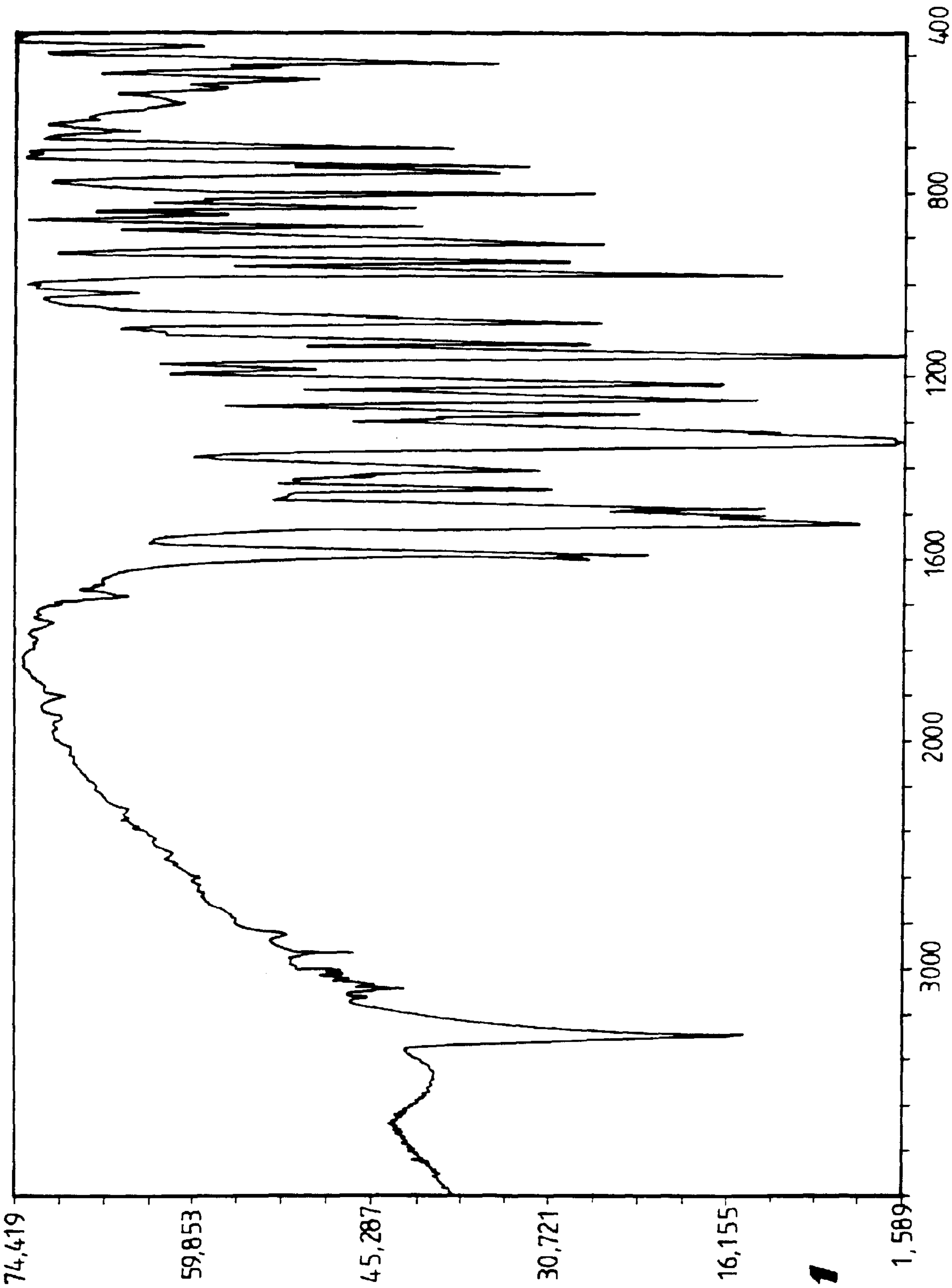
74,419
59,853
45,287
30,721
16,155
1,589
400
800
1200
1600
2000
3000

Fig.1

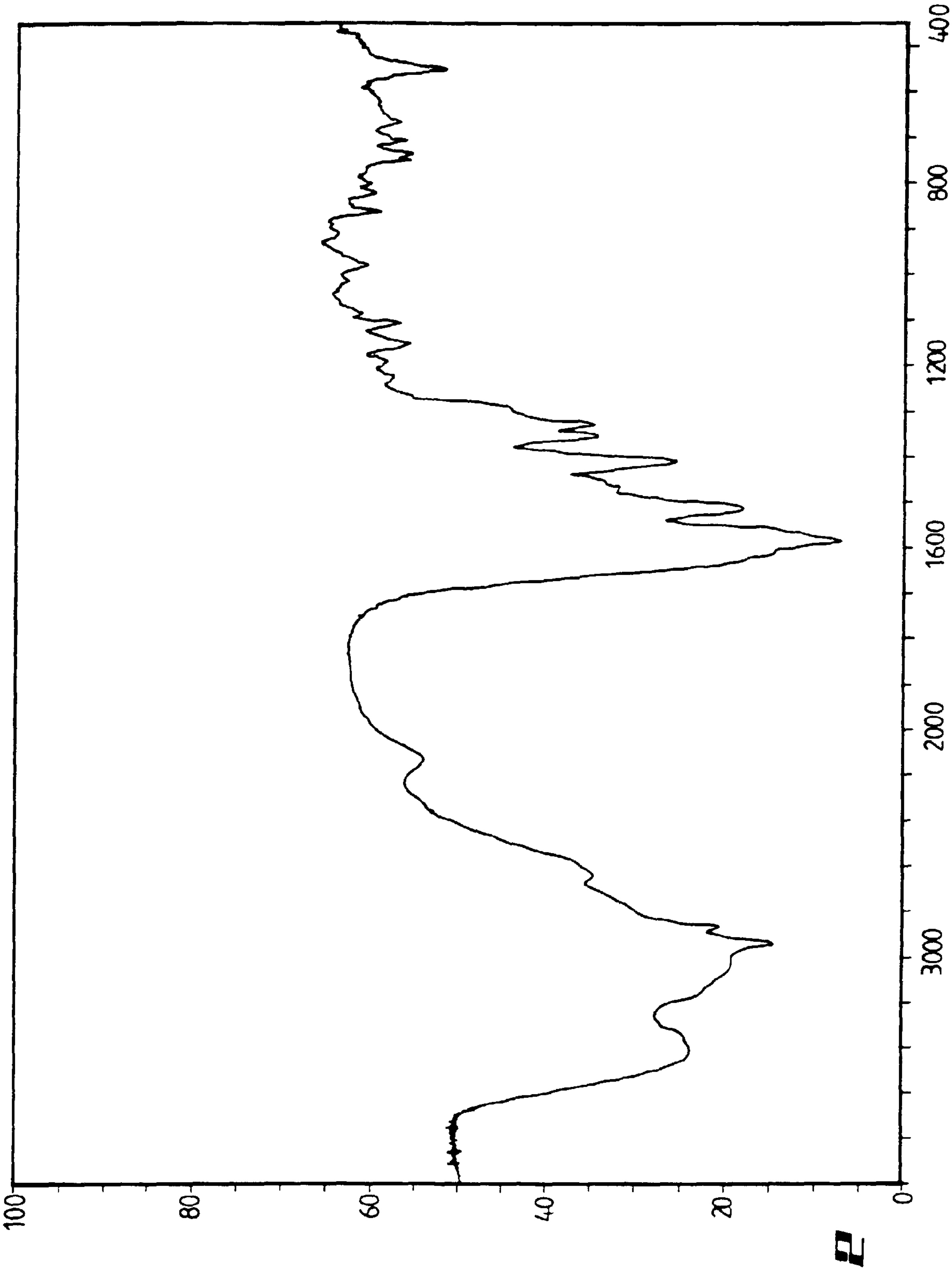


Fig.2

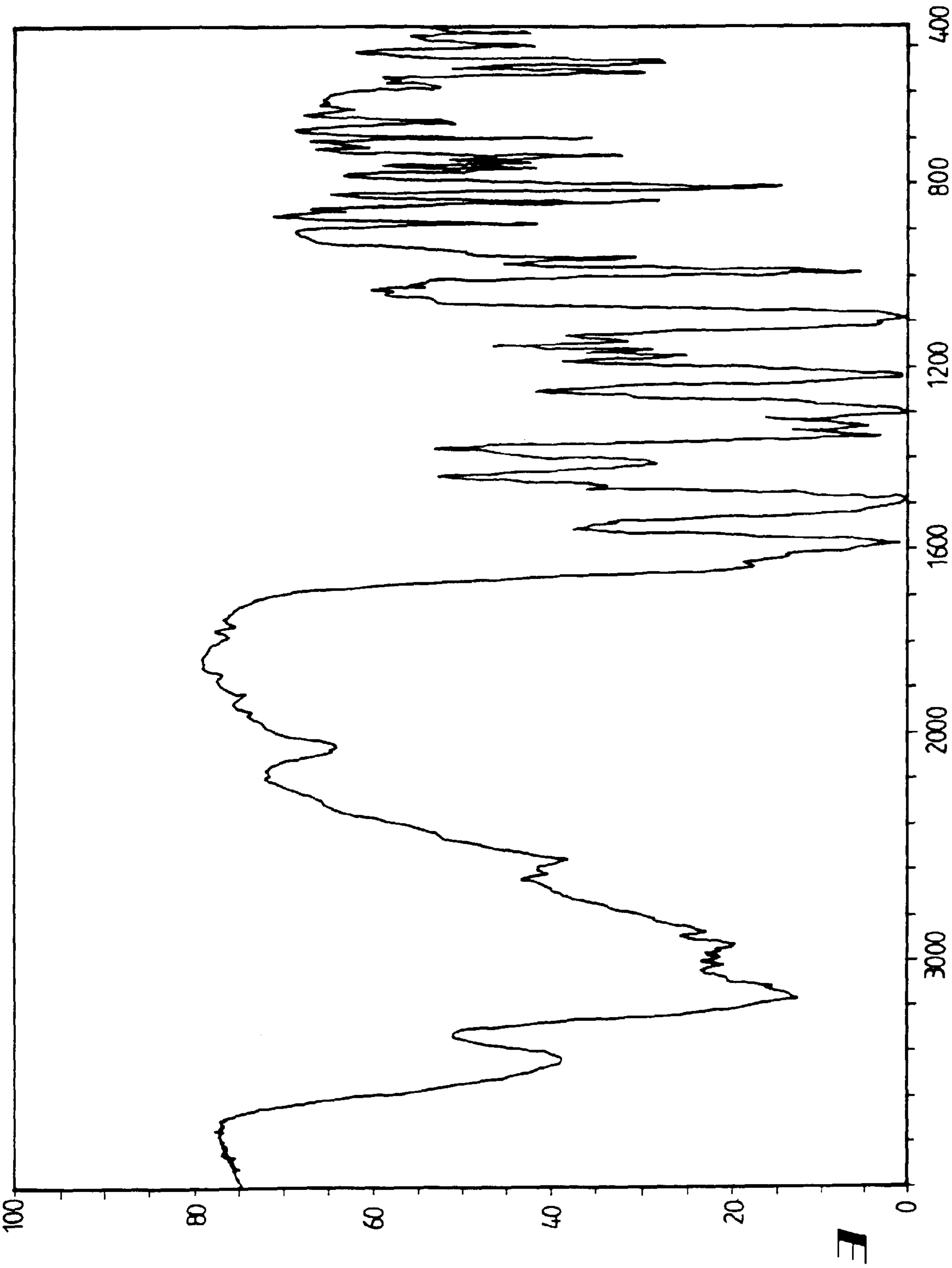


Fig. 3

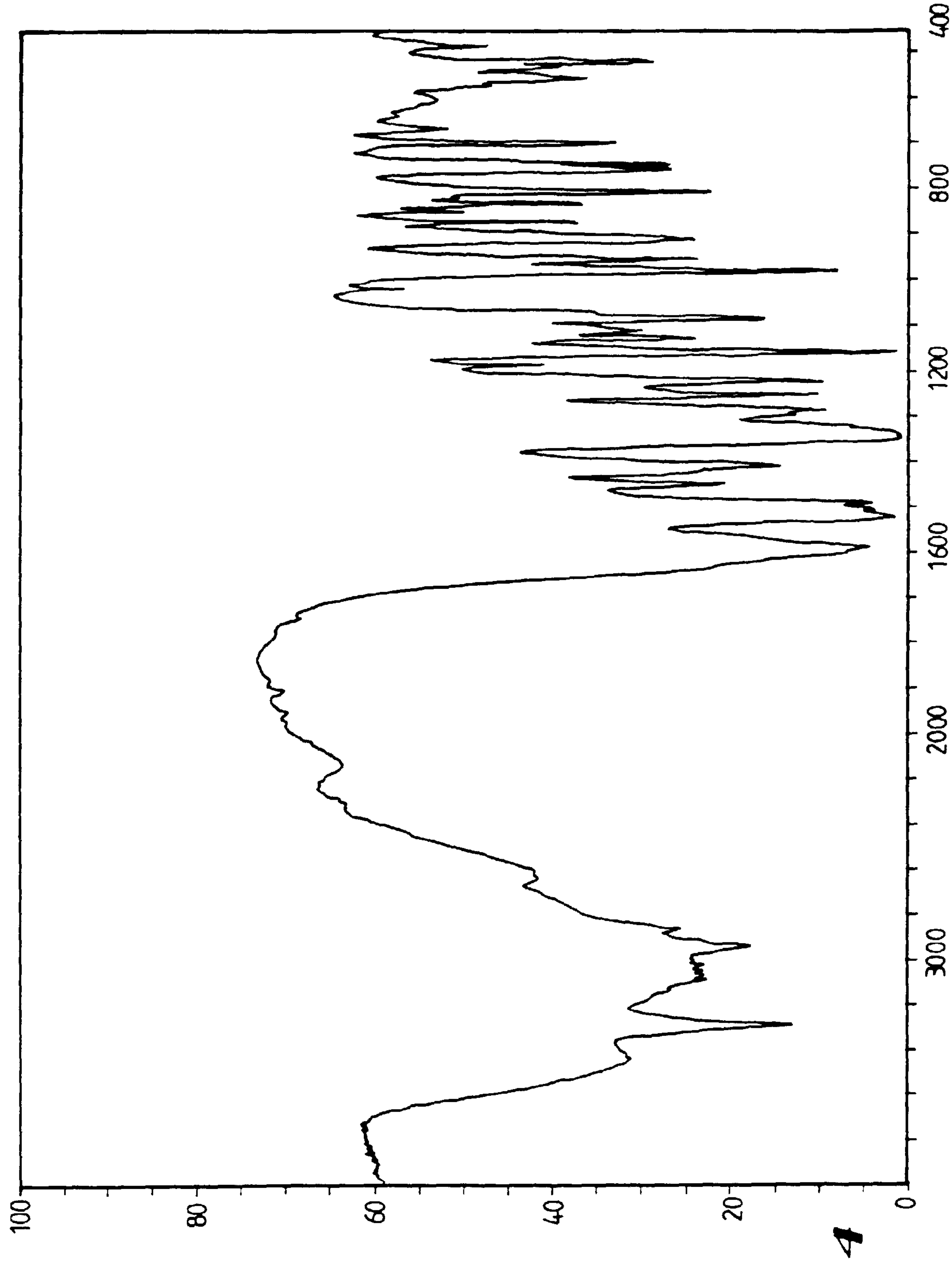


Fig.4

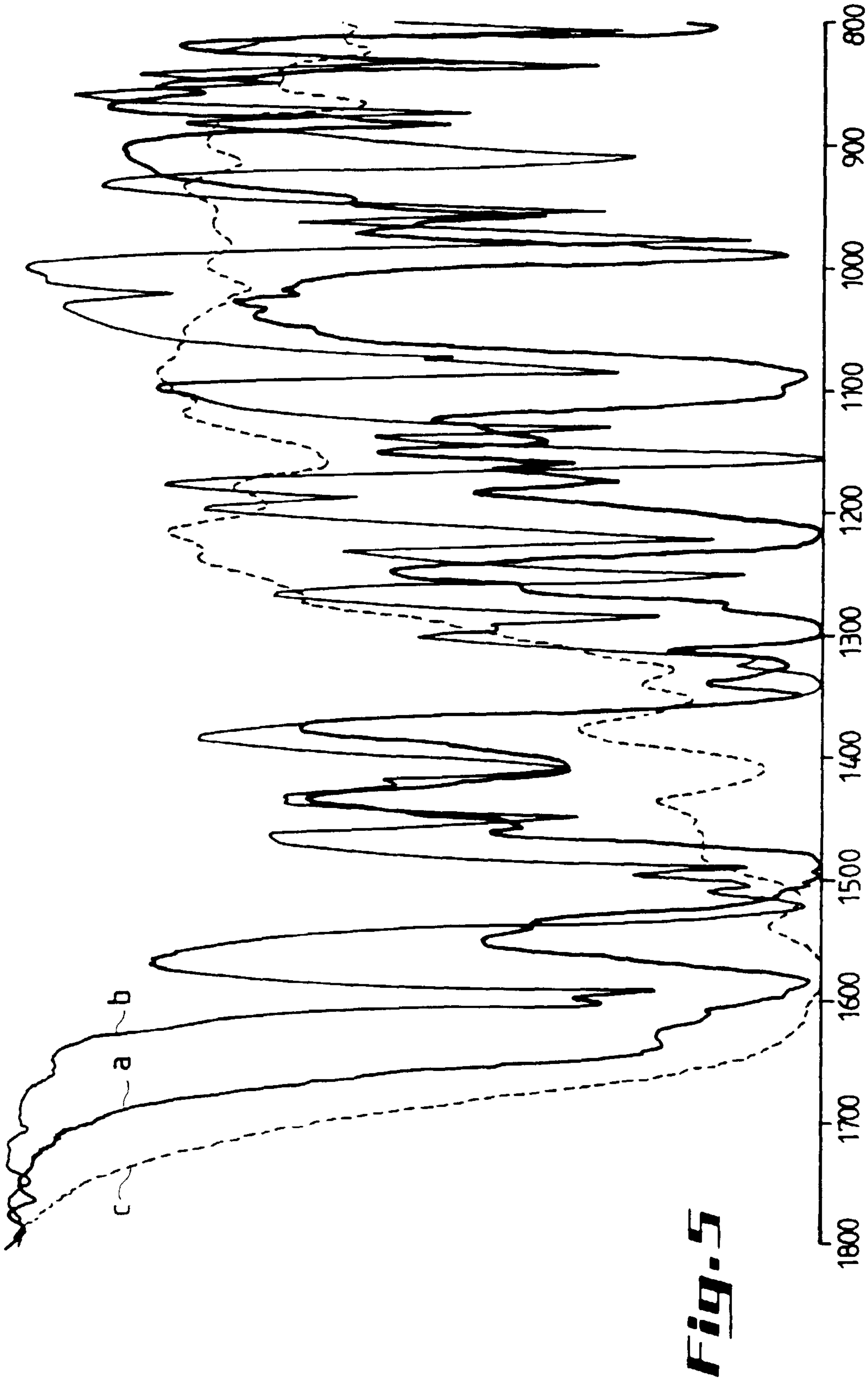


Fig. 5

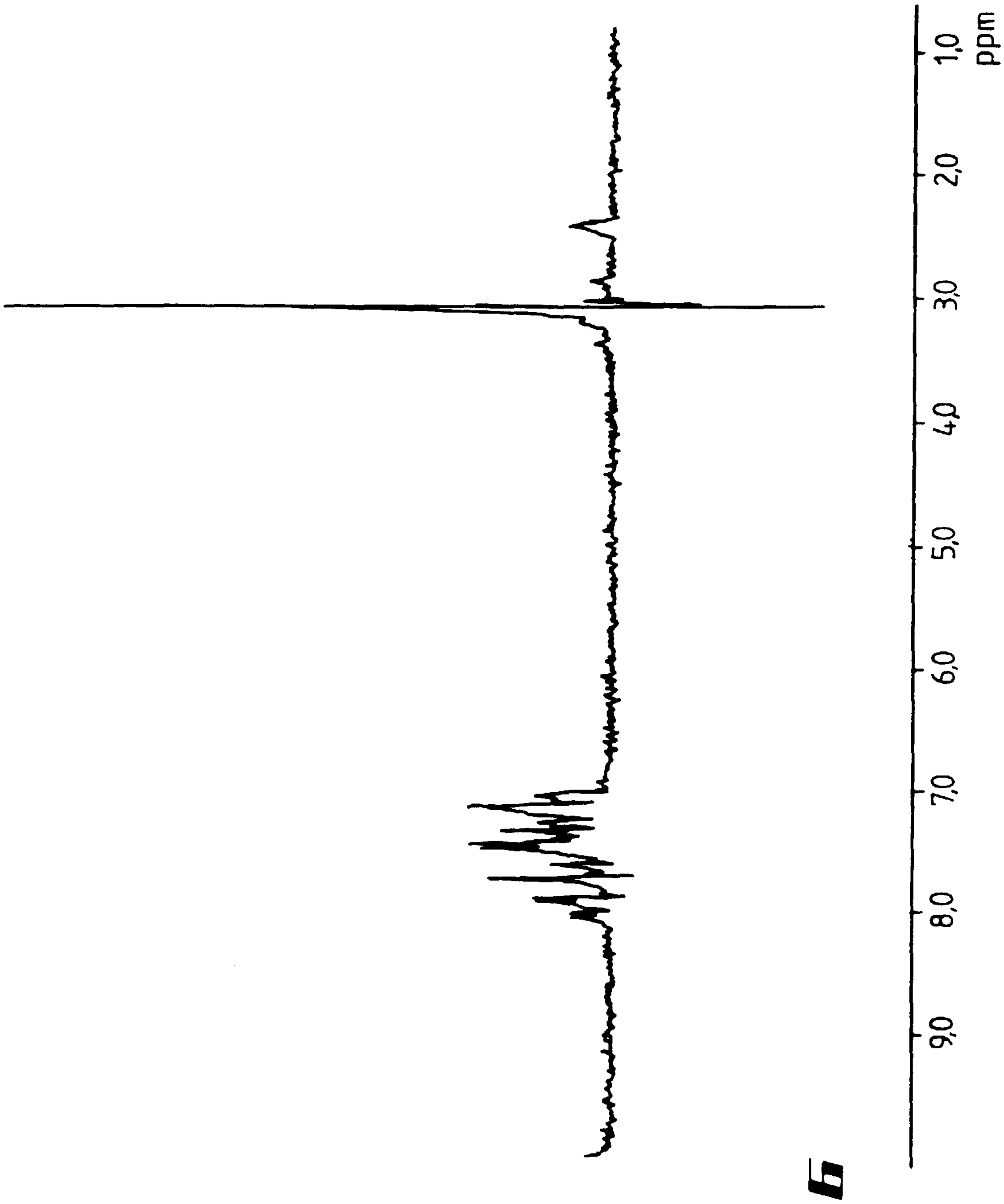


Fig.6

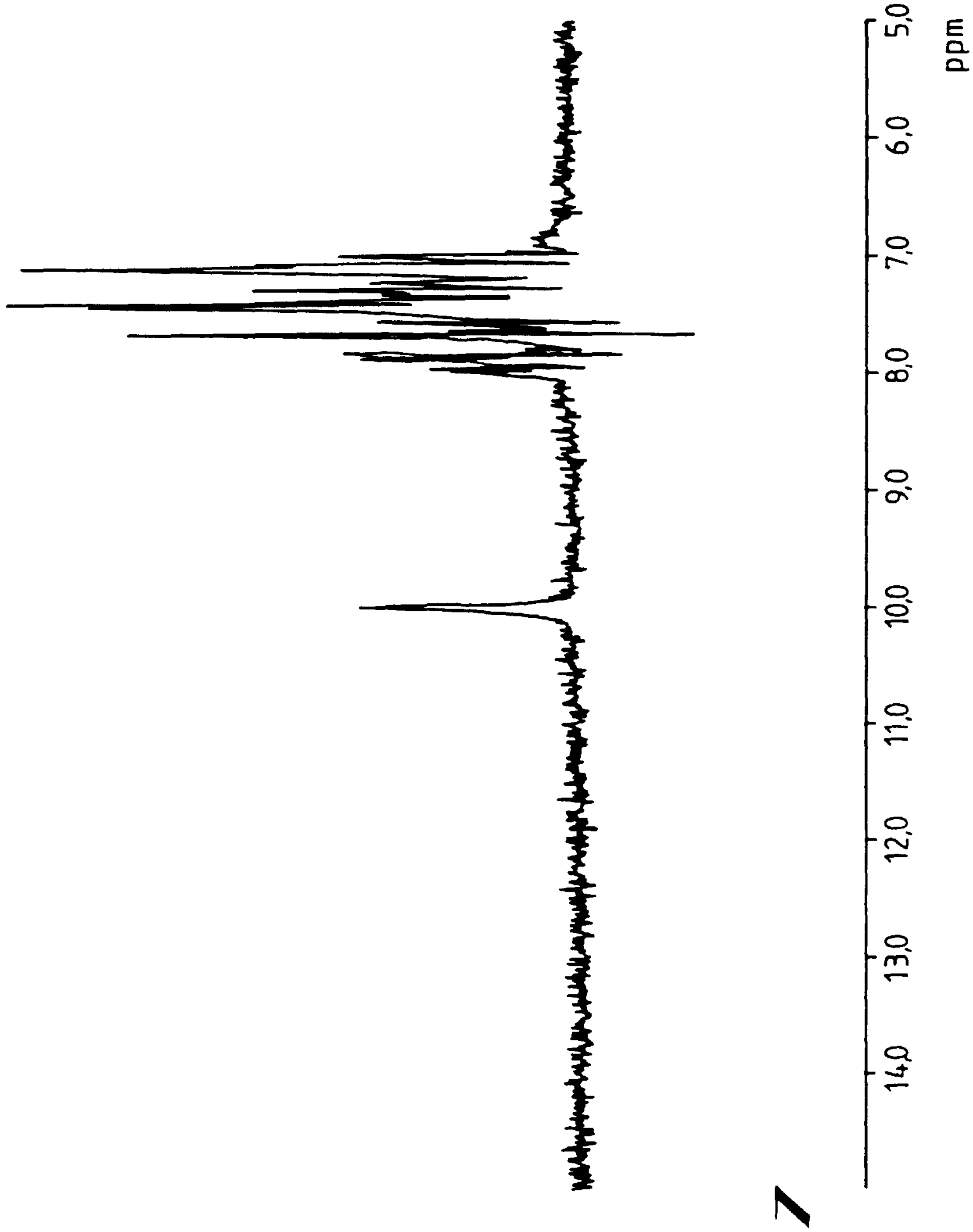


Fig.7

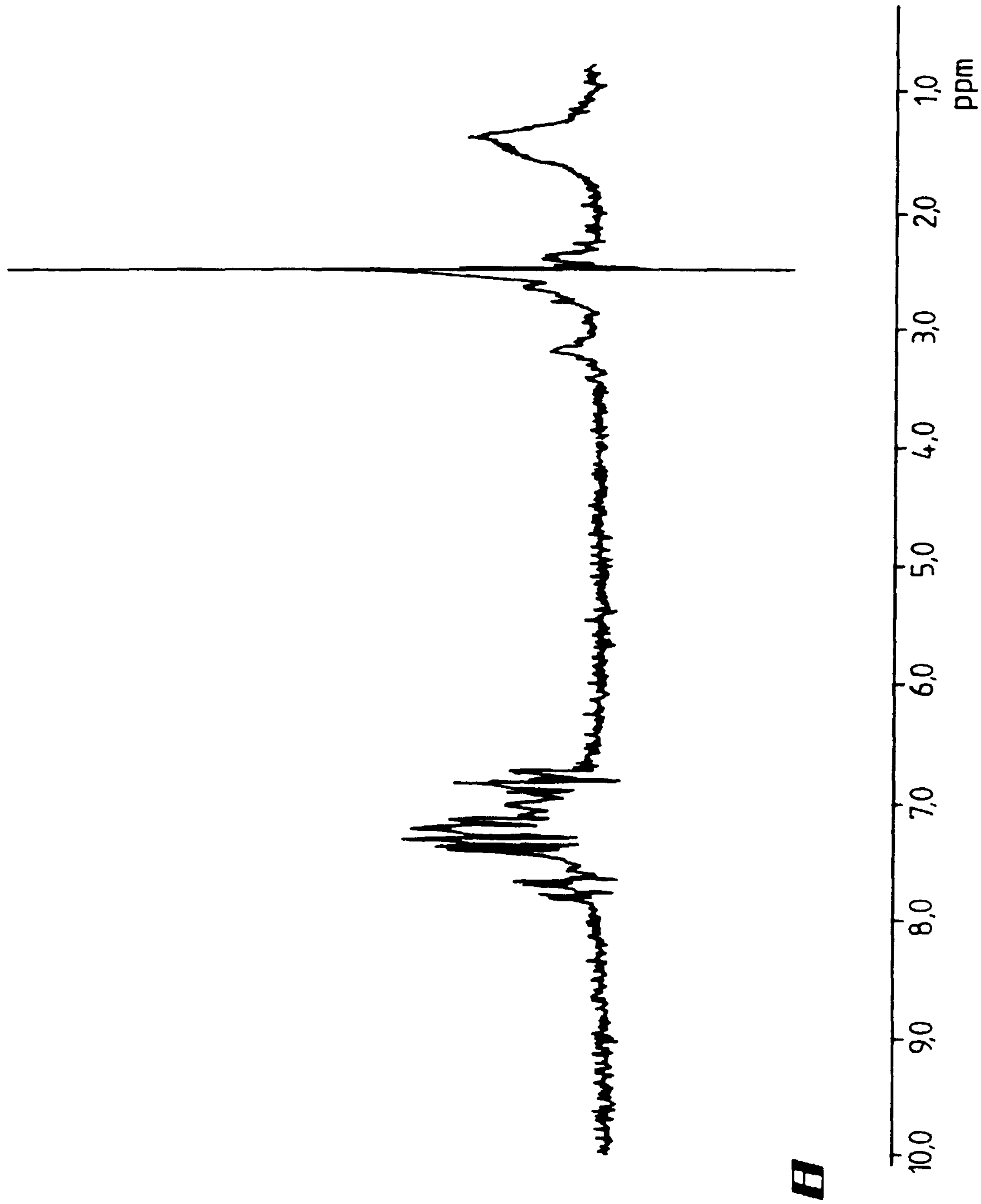


Fig. 8

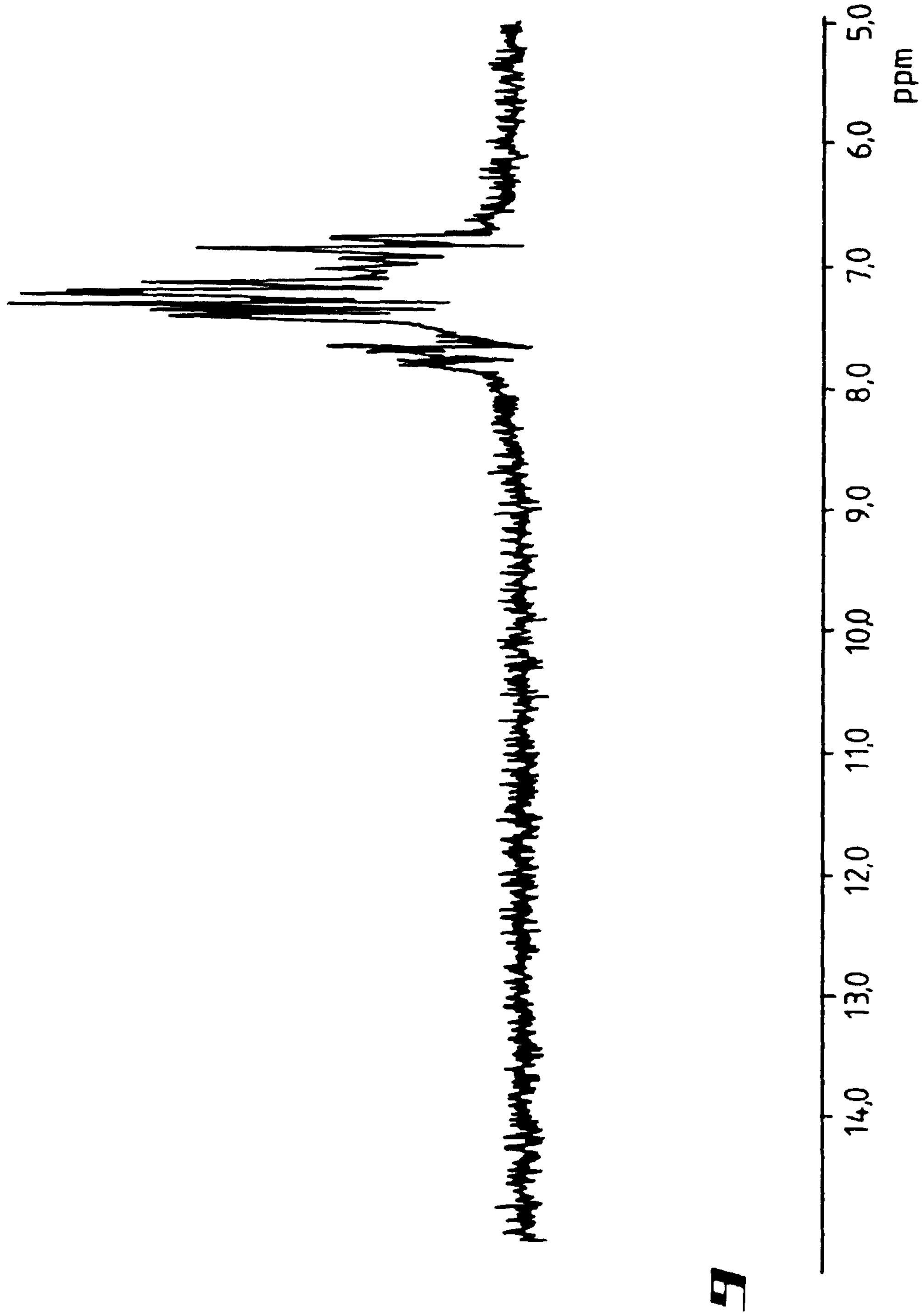


Fig.9

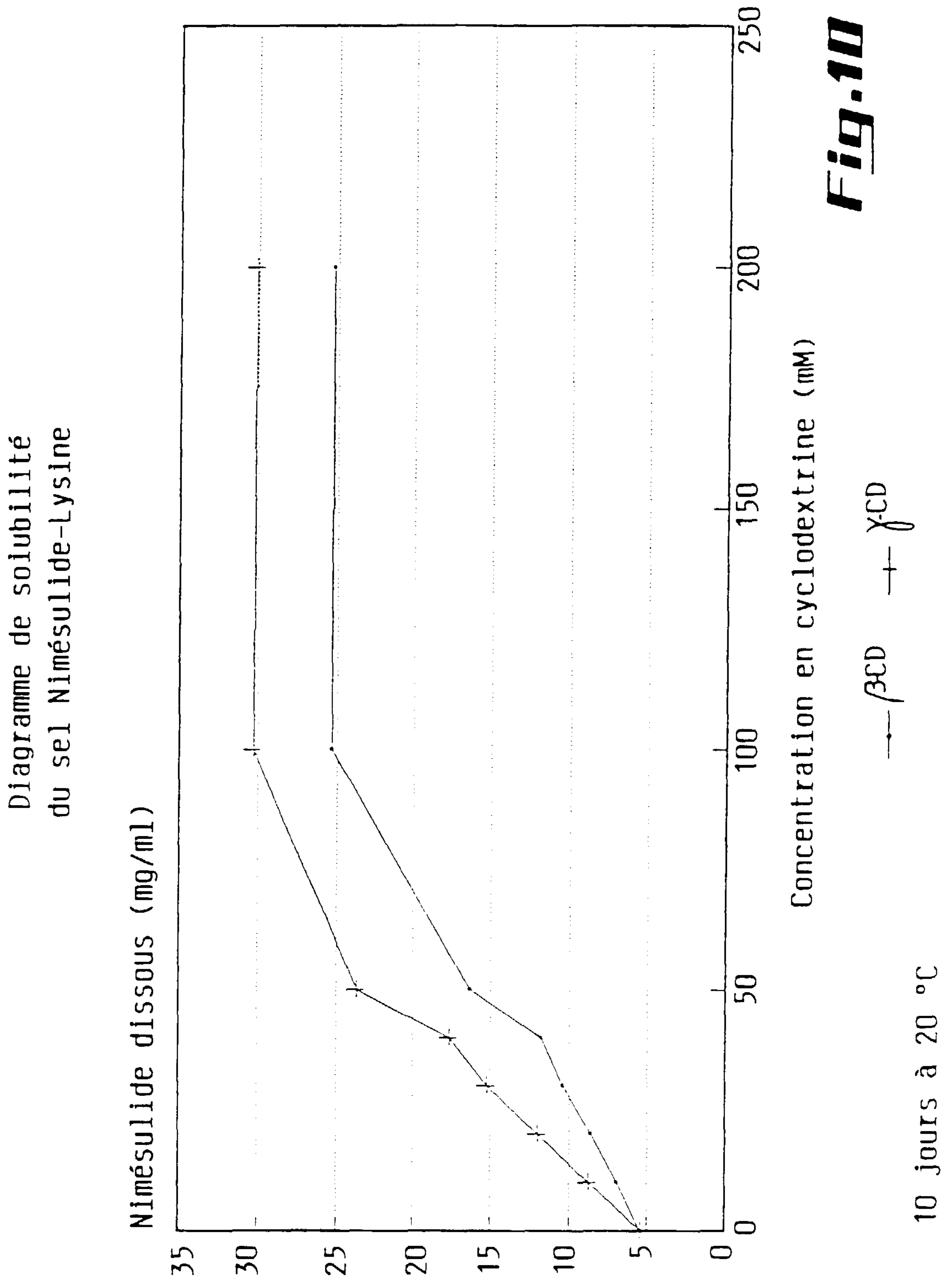
Diagramme de solubilité
du sel Nimésulide-Lysine
Nimésulide dissous (mg/ml)
35
30
25
20
15
10
5
0
0
50
100
150
200
250
Concentration en cyclodextrine (mM)
βCD
γCD
10 jours à 20 °C

Fig.10

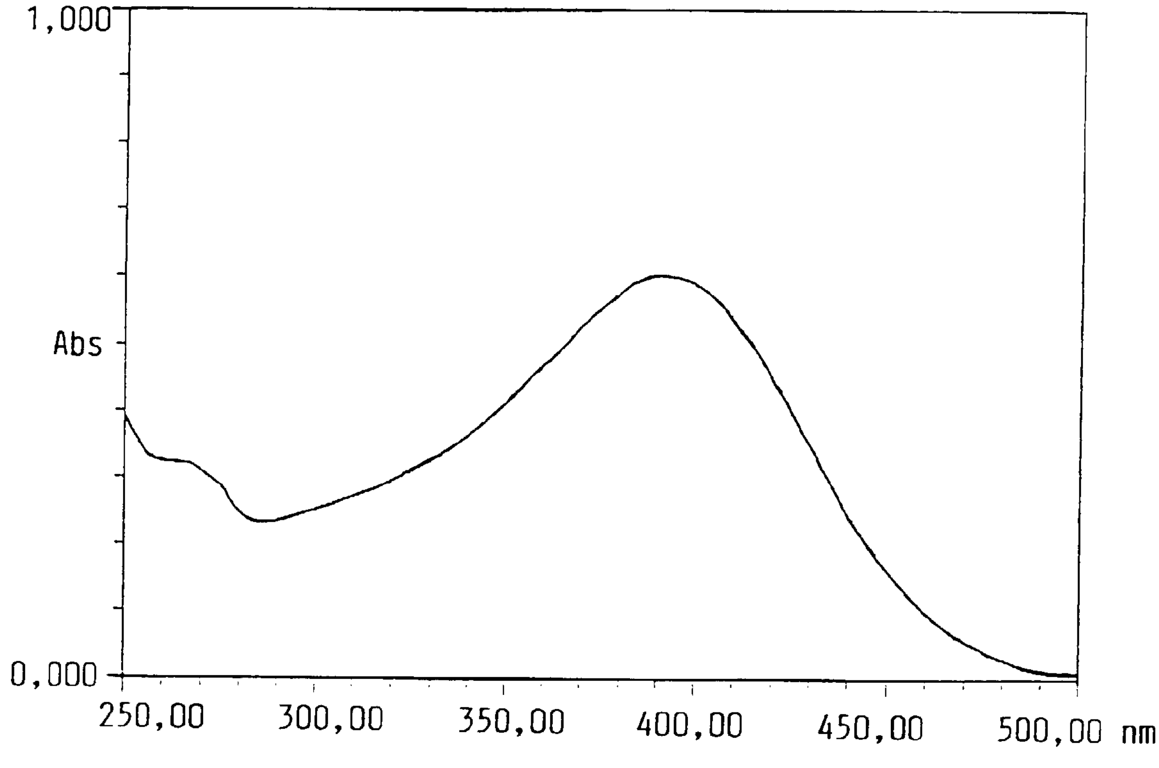


Fig.11

Thermogramme du complexe nimésulide-L-lysine-βCD obtenu par atomisation.

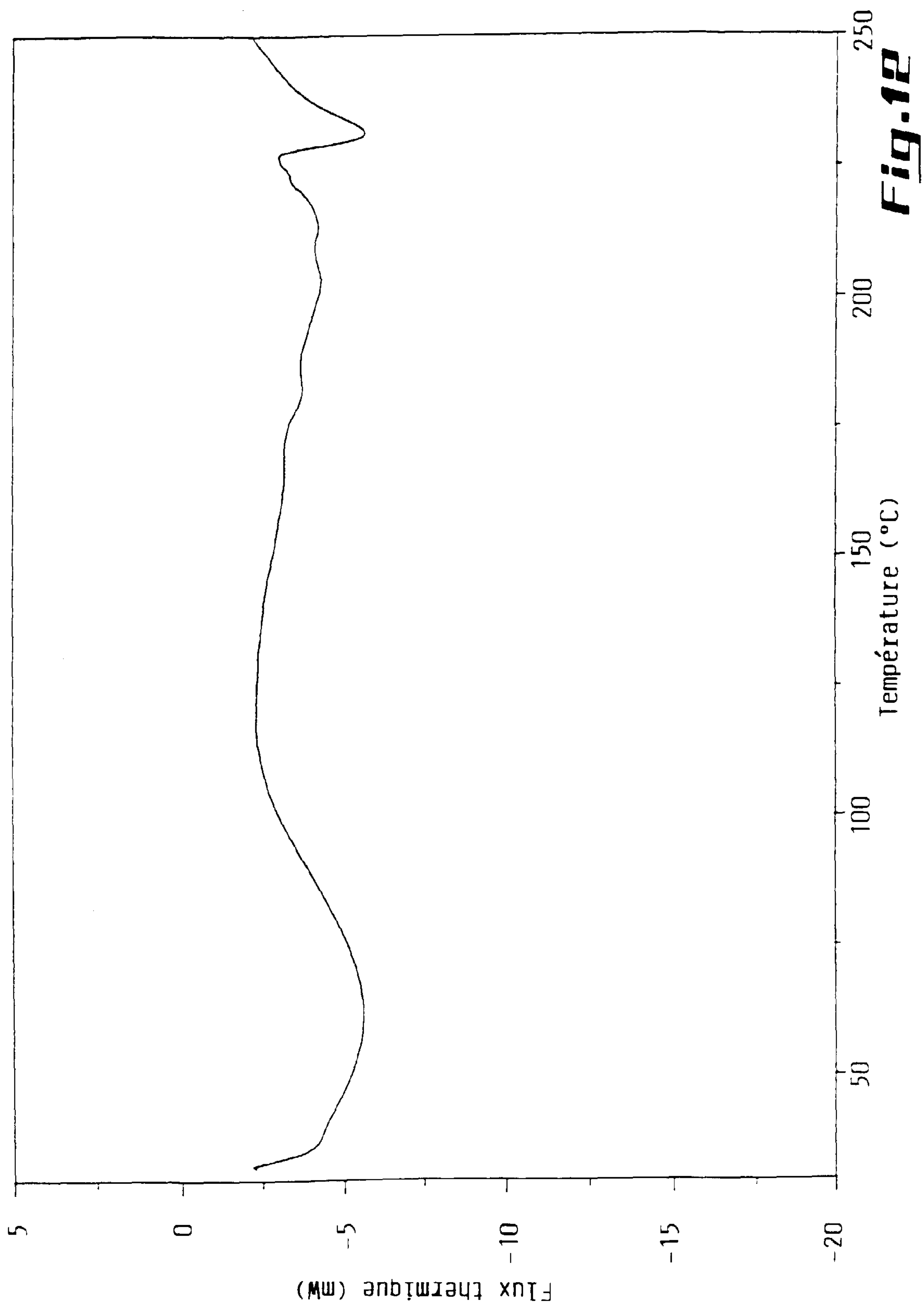


Fig.12

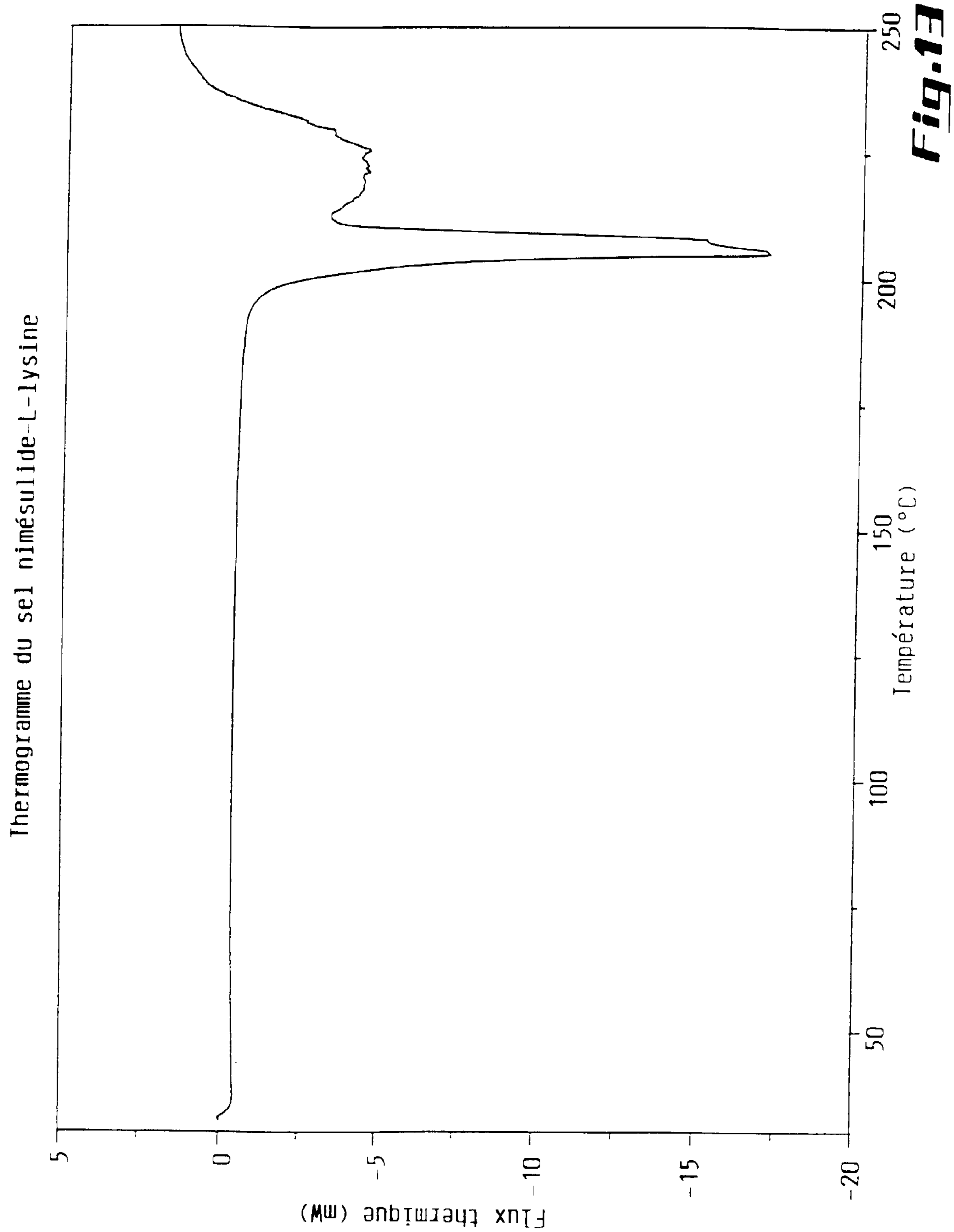
Thermogramme du sel nimésulide-L-lysine
Flux thermique (mW)
5
0
-5
-10
-15
-20
Température (°C)
50
100
150
200
250

Fig.13

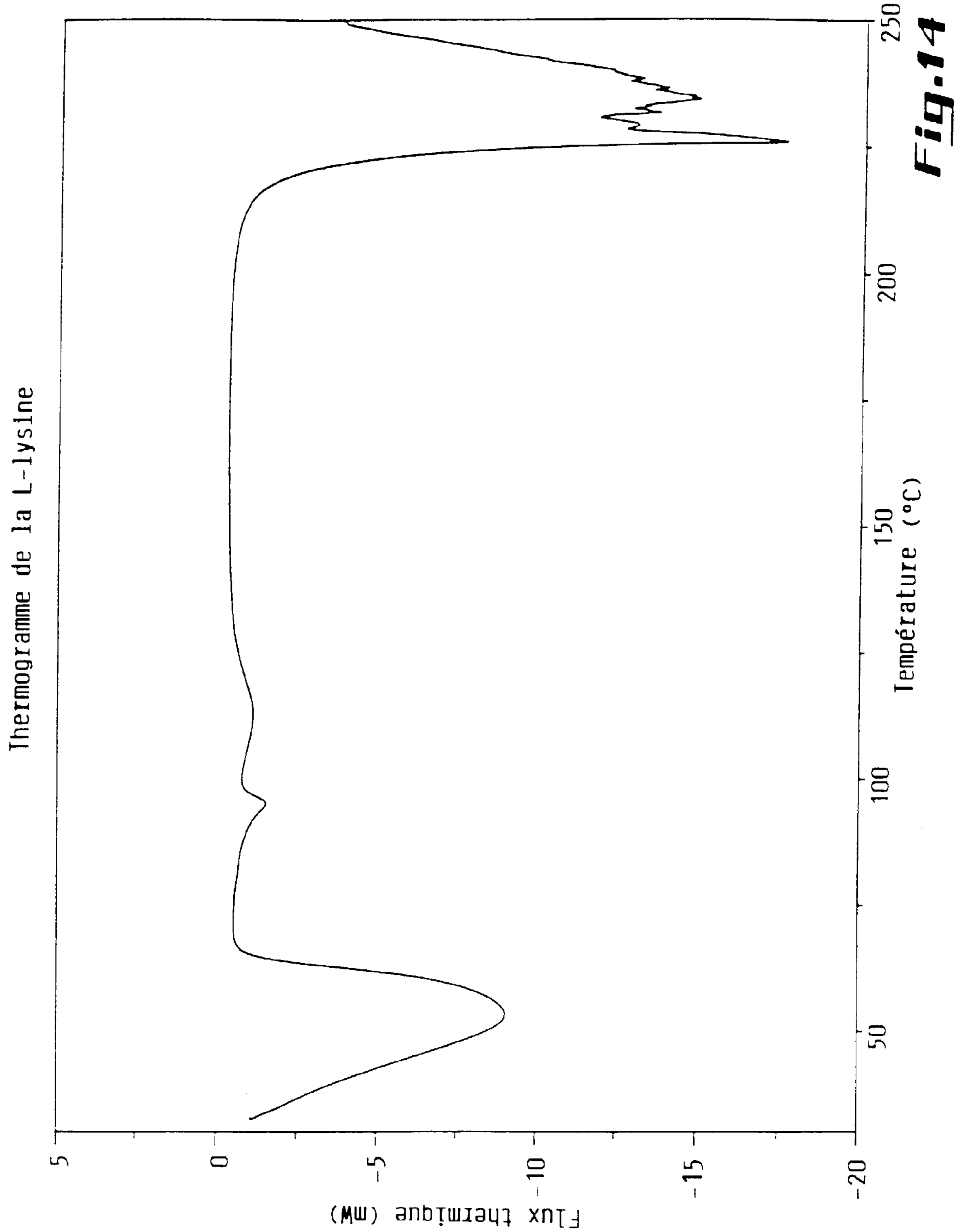
Thermogramme de la L-lysine
Flux thermique (mW)
5
0
-5
-10
-15
-20
Température (°C)
50
100
150
200
250

Fig.14

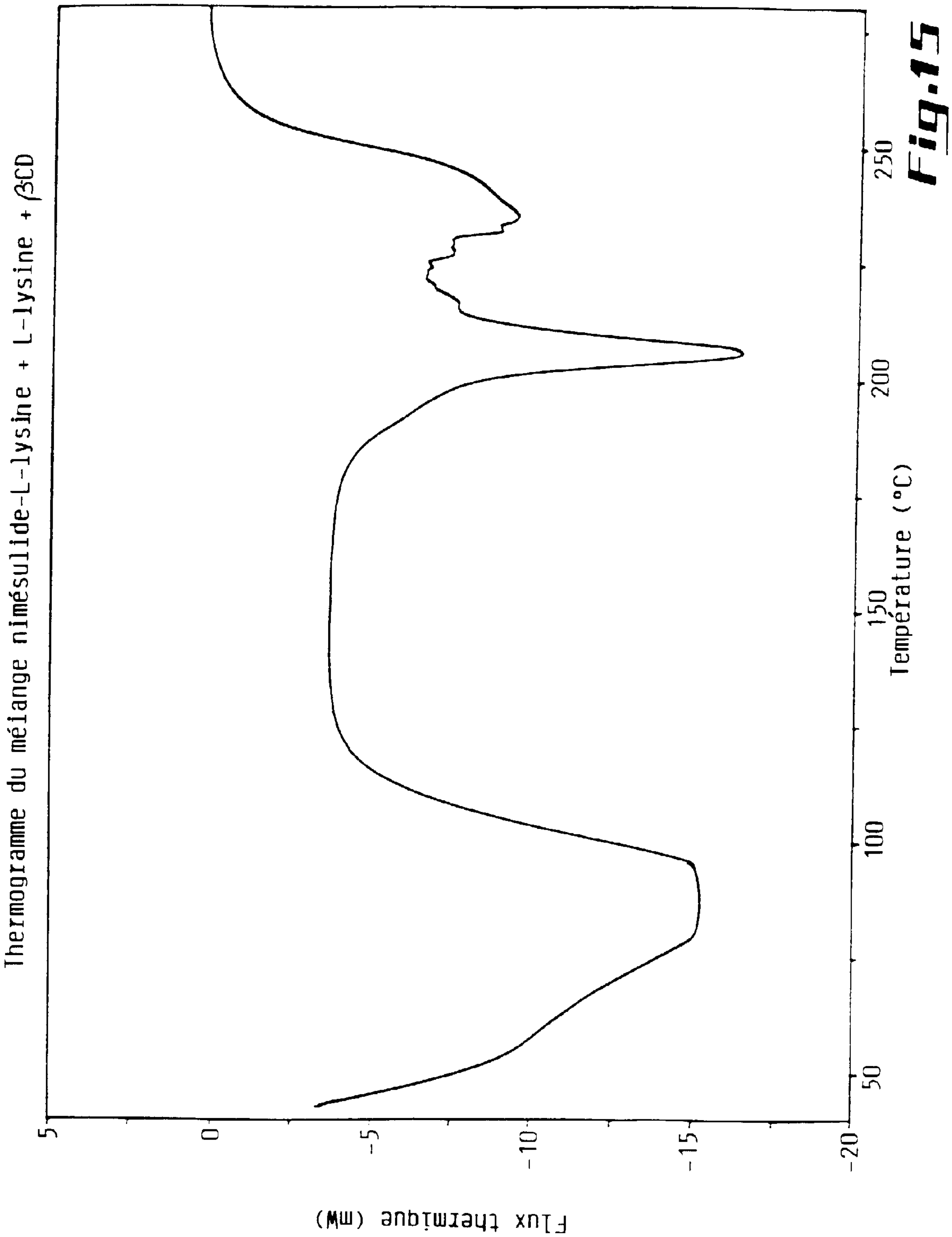
Thermogramme du mélange nimésulide-L-lysine + L-lysine + βCD
Flux thermique (mW)
5
0
-5
-10
-15
-20
50
100
150
200
250
Température (°C)

**Fig.15**

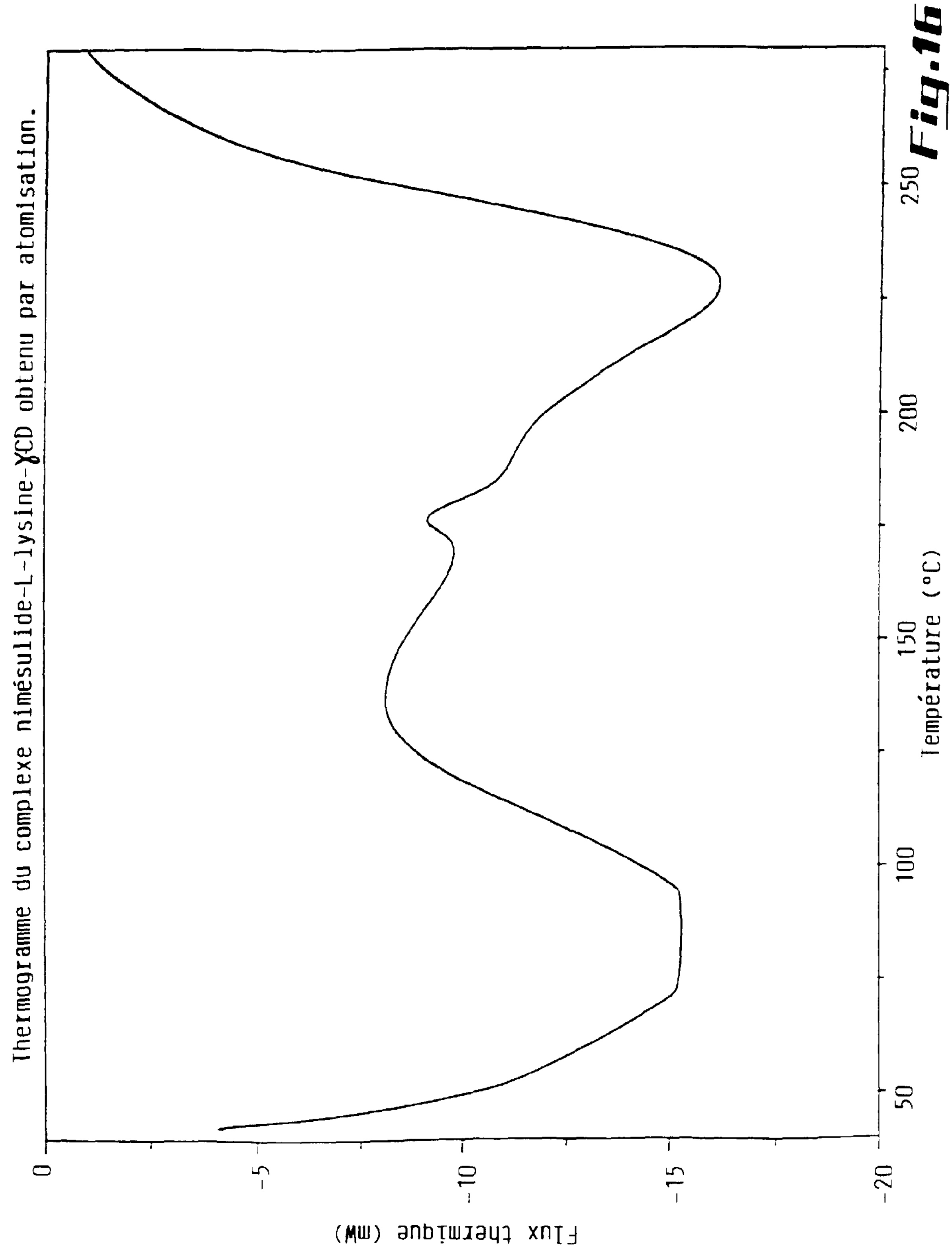
Thermogramme du complexe nimésulide-L-lysine-γCD obtenu par atomisation.
0
-5
-10
-15
-20
Flux thermique (mW)
50
100
150
200
250
Température (°C)

Fig.16

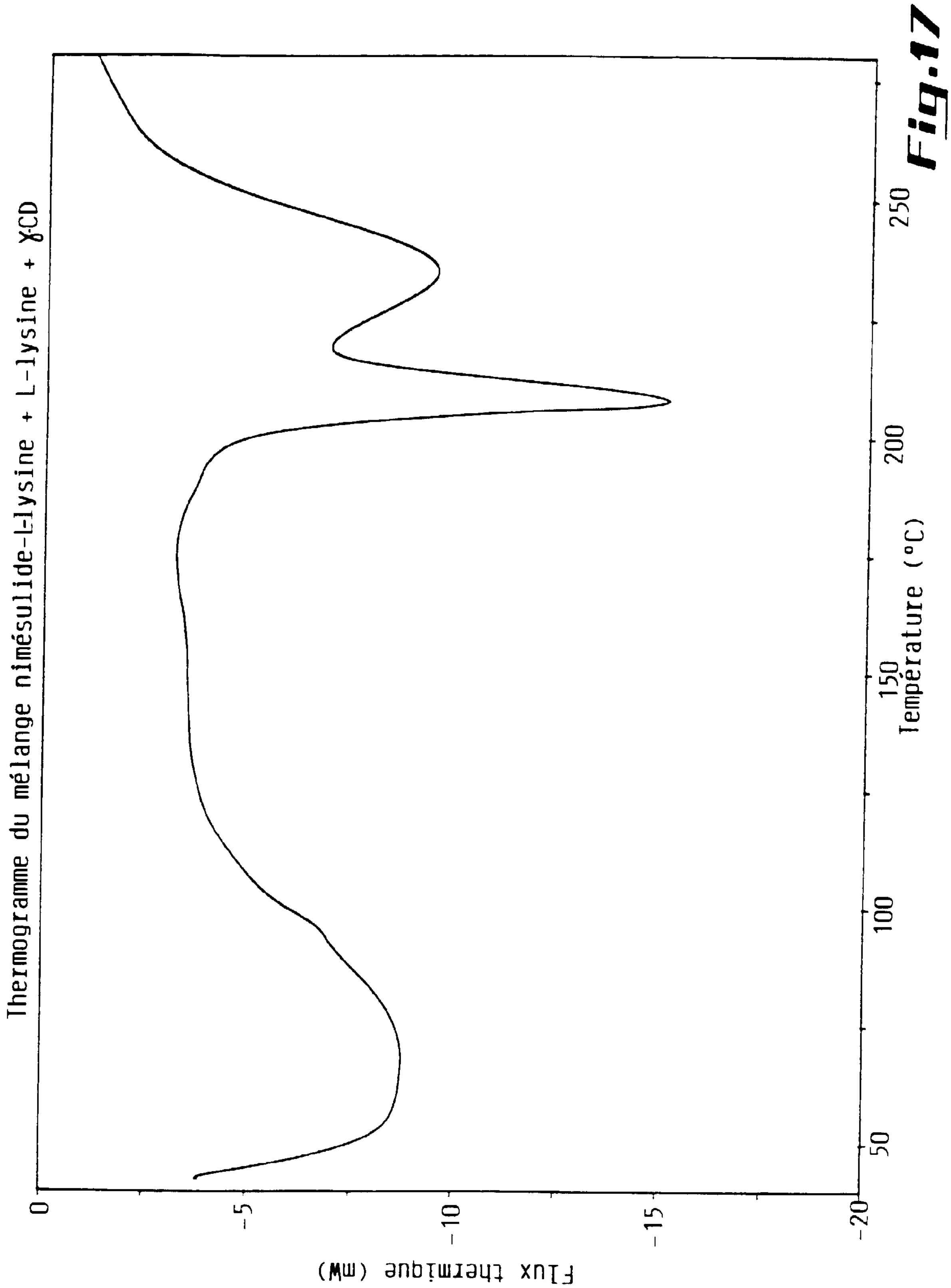
Thermogramme du mélange nimésulide-L-lysine + L-lysine + γCD
Flux thermique (mW)
0
-5
-10
-15
-20
50
100
150
200
250
Température (°C)

Fig.17

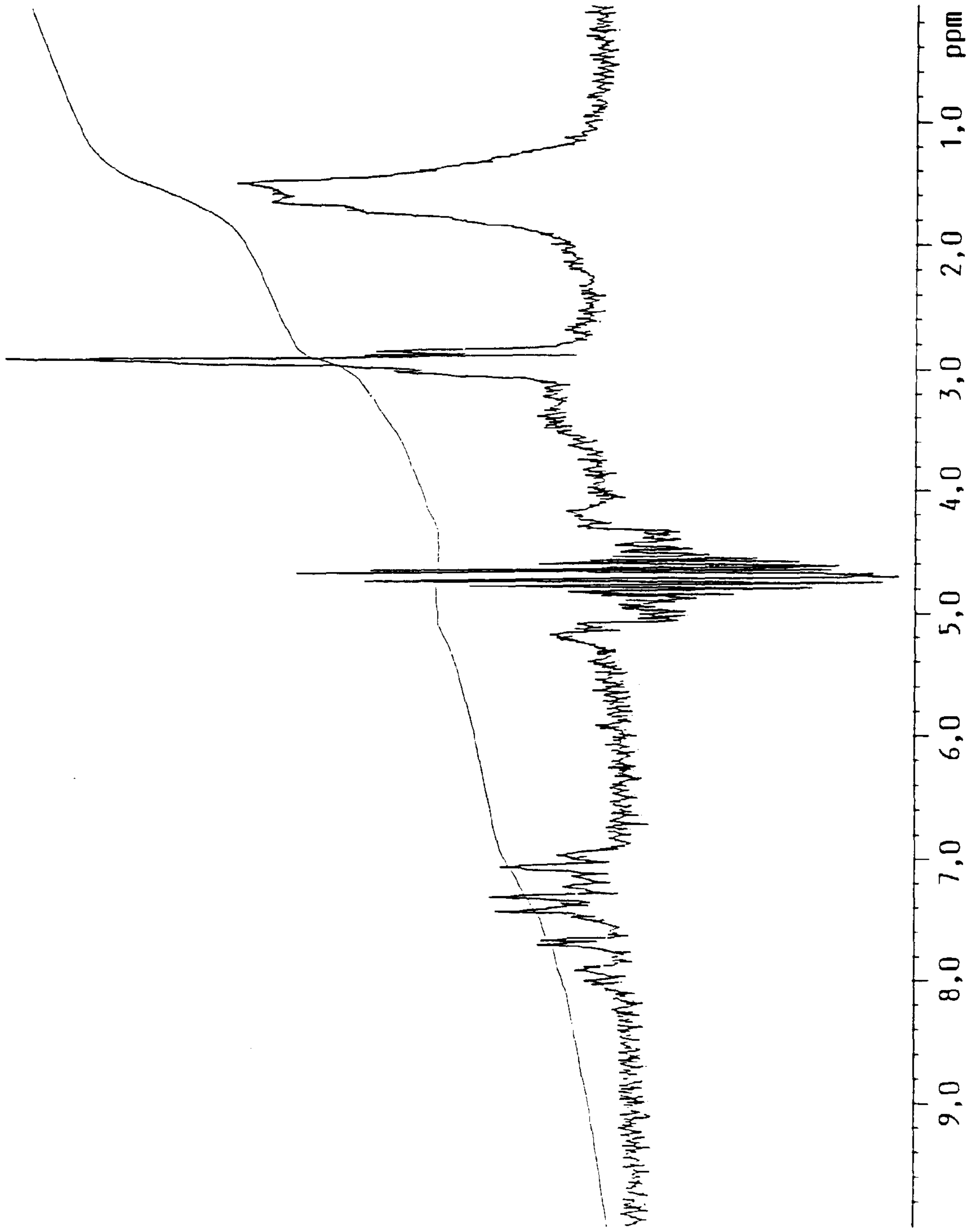


Fig.18

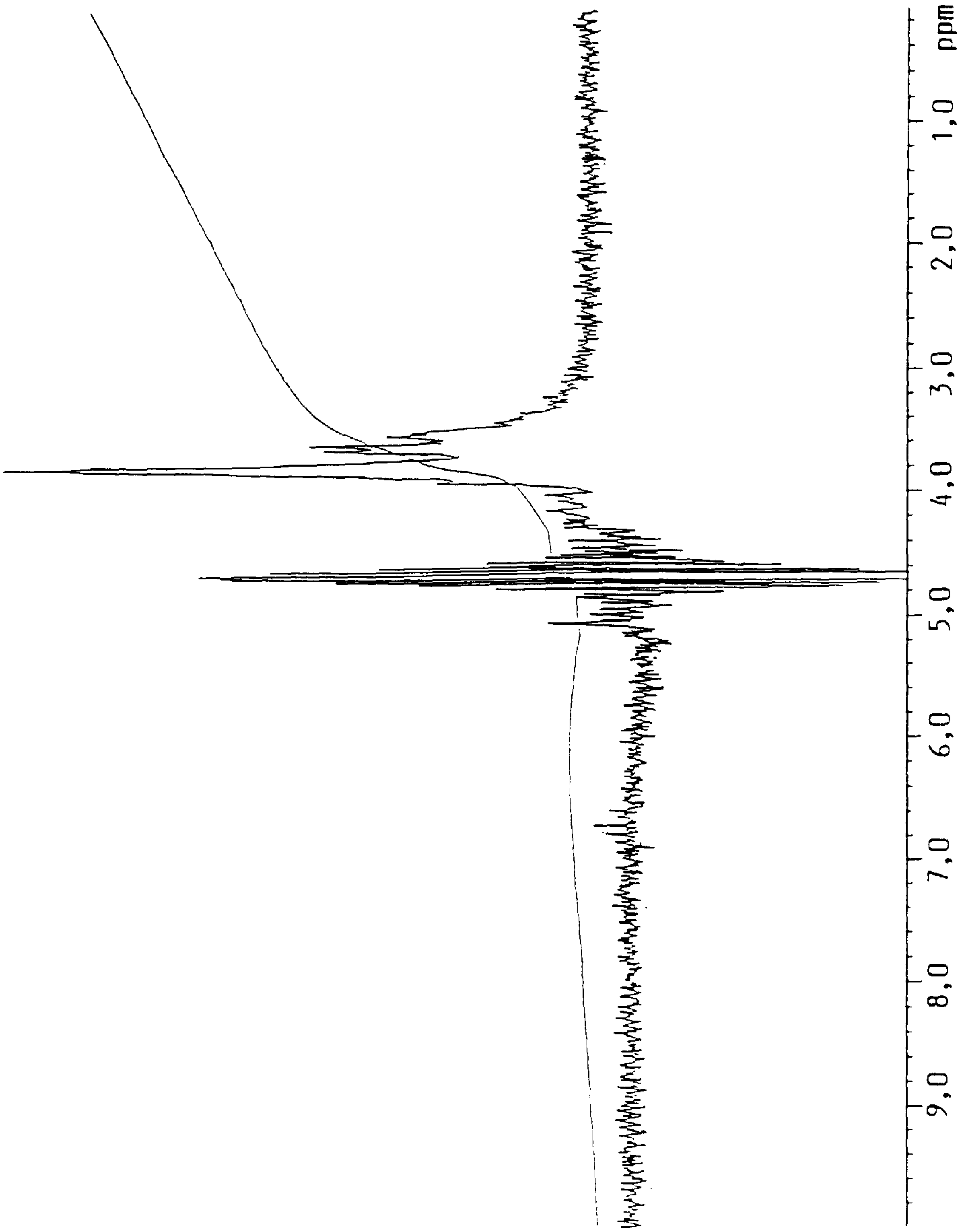


Fig.19

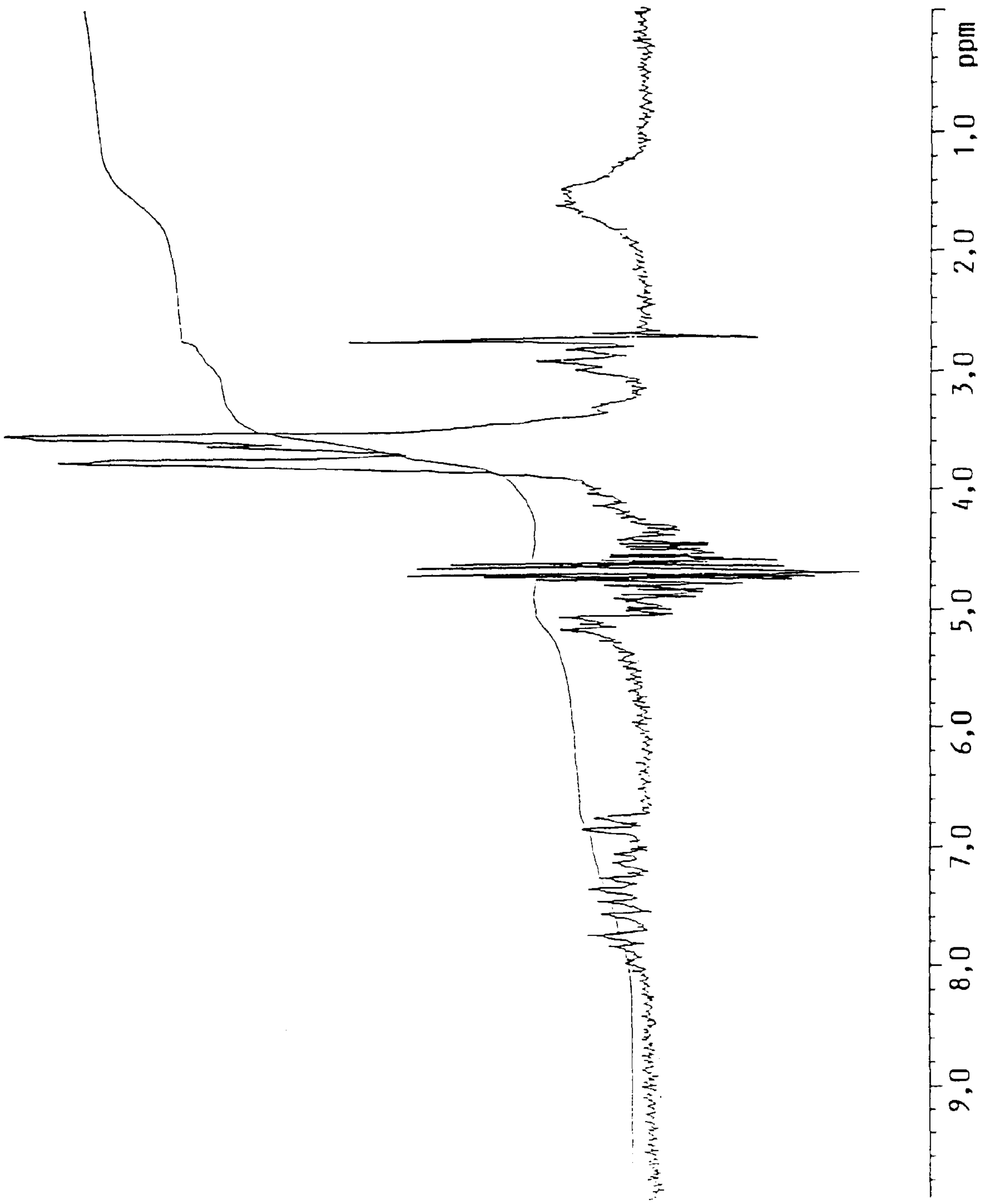


Fig. 20

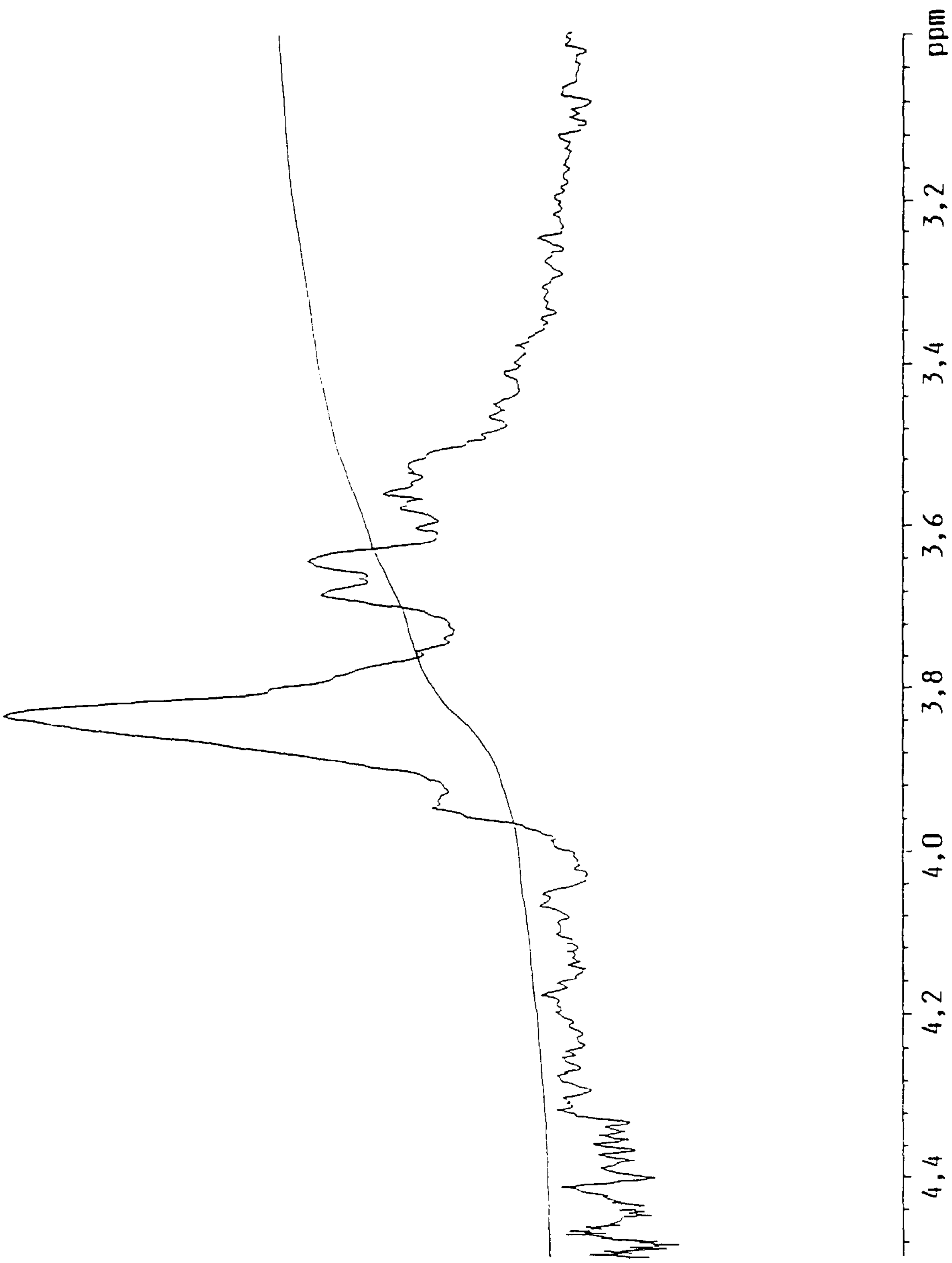


Fig.21

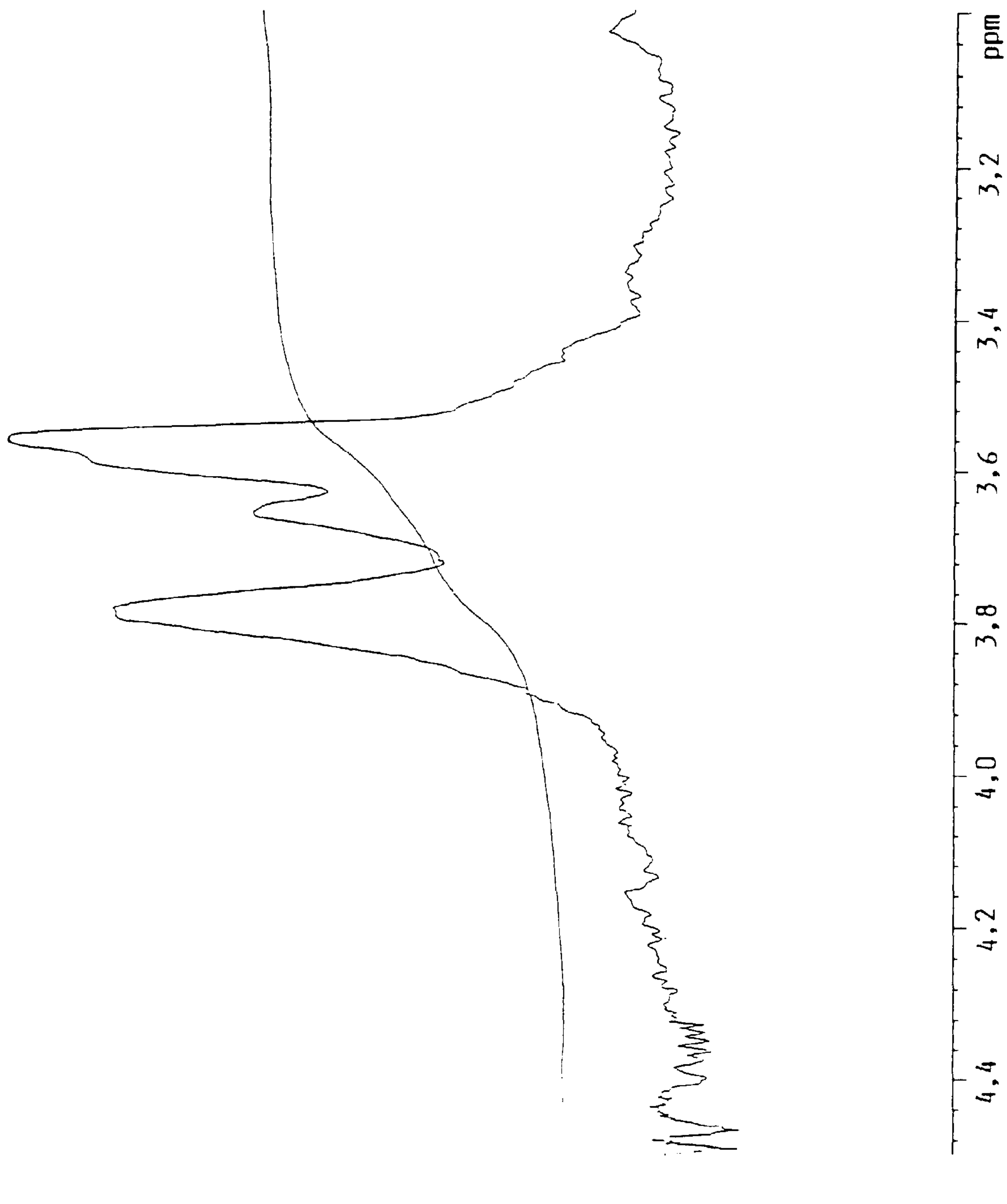


Fig.22

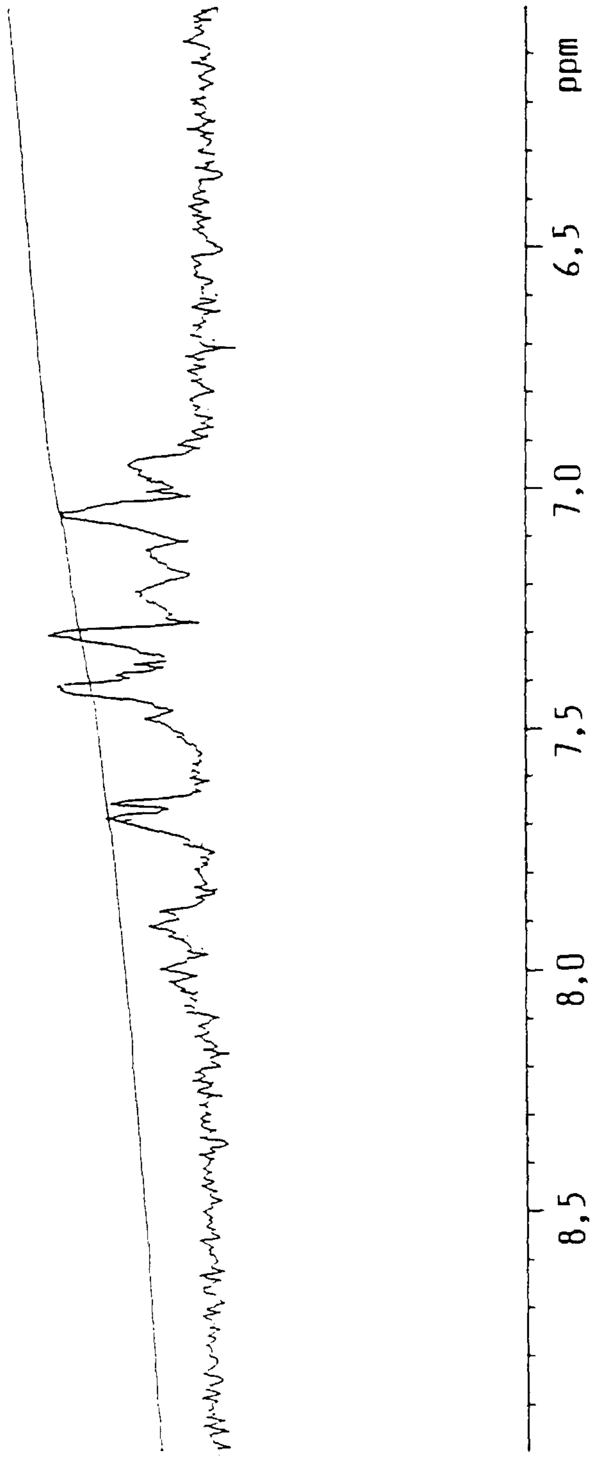


Fig.23

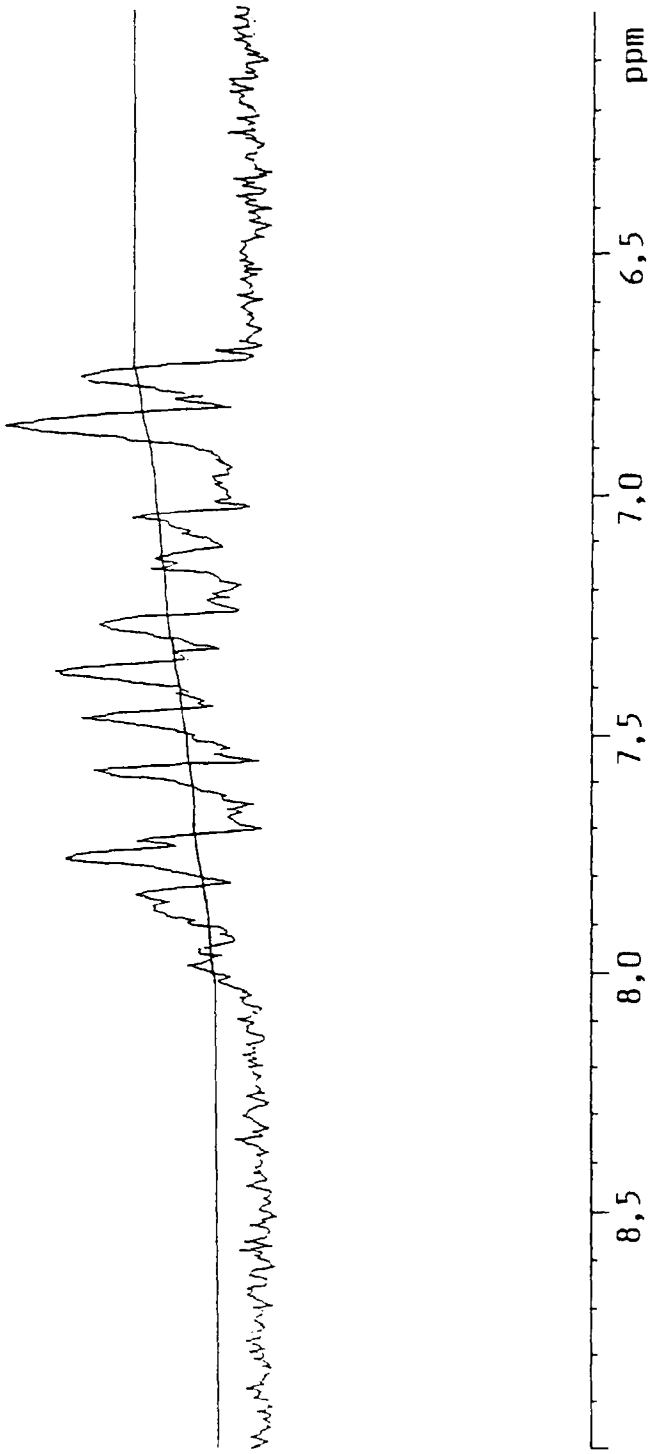


Fig.24

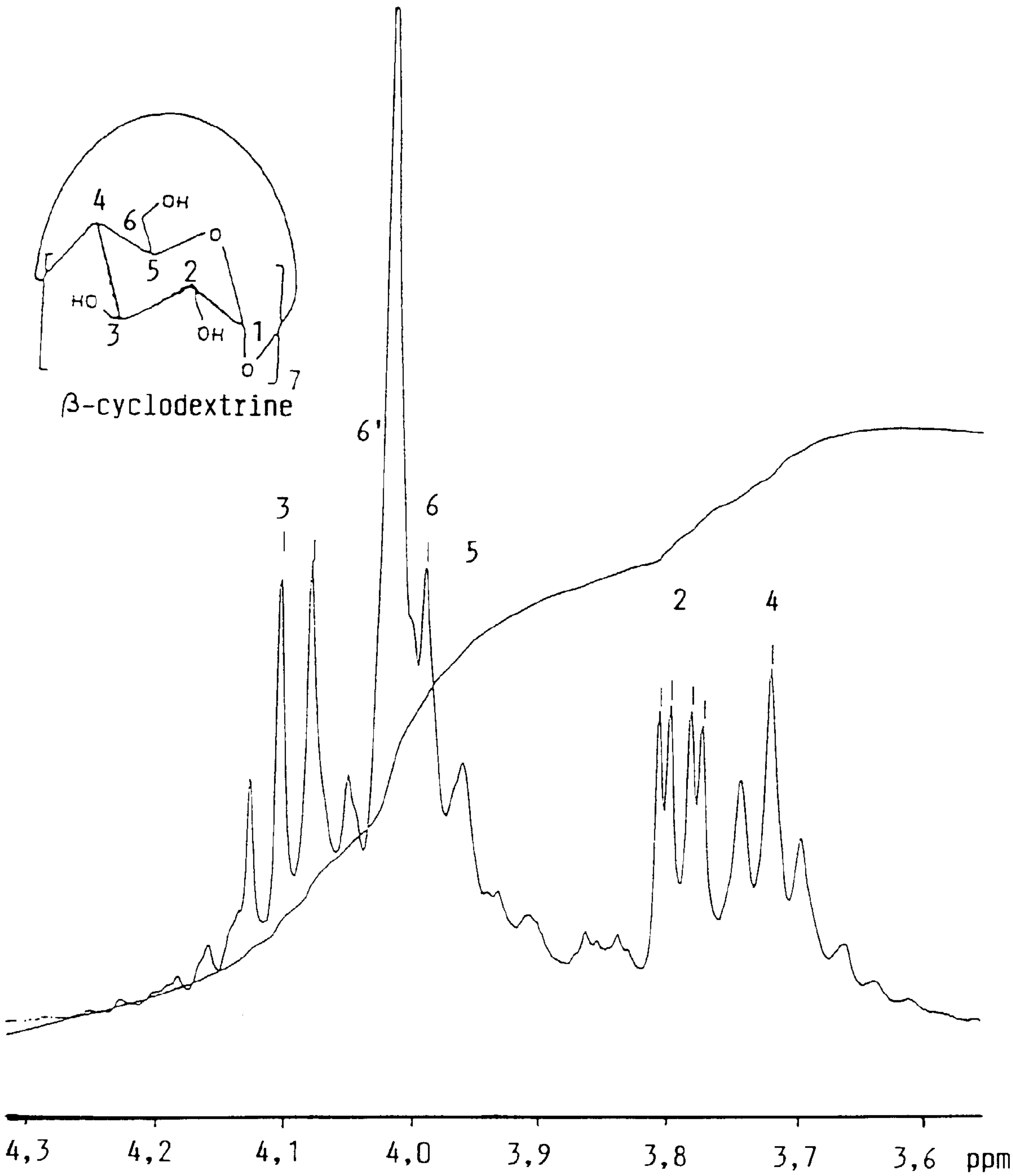


Fig.25

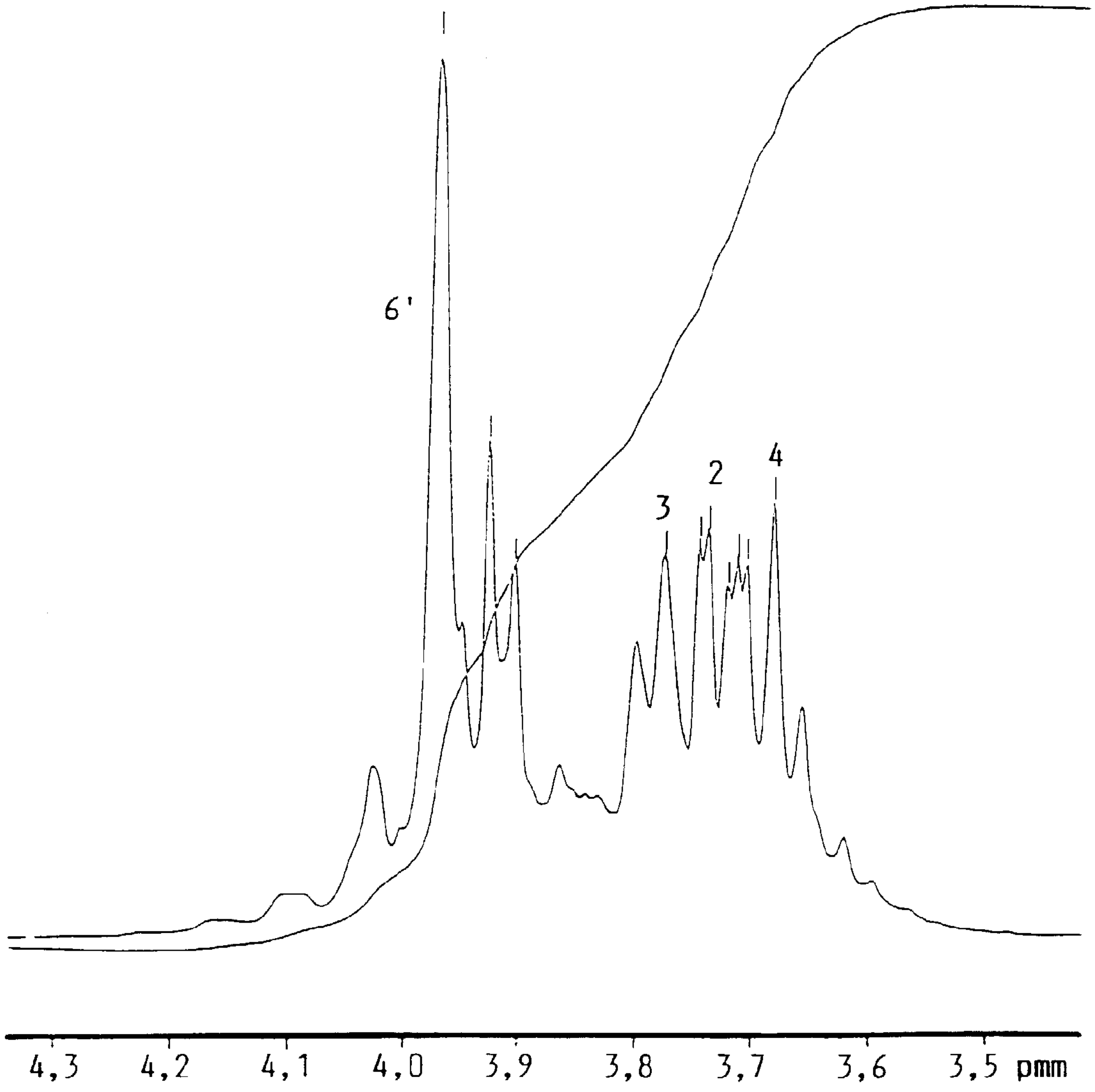


Fig.26

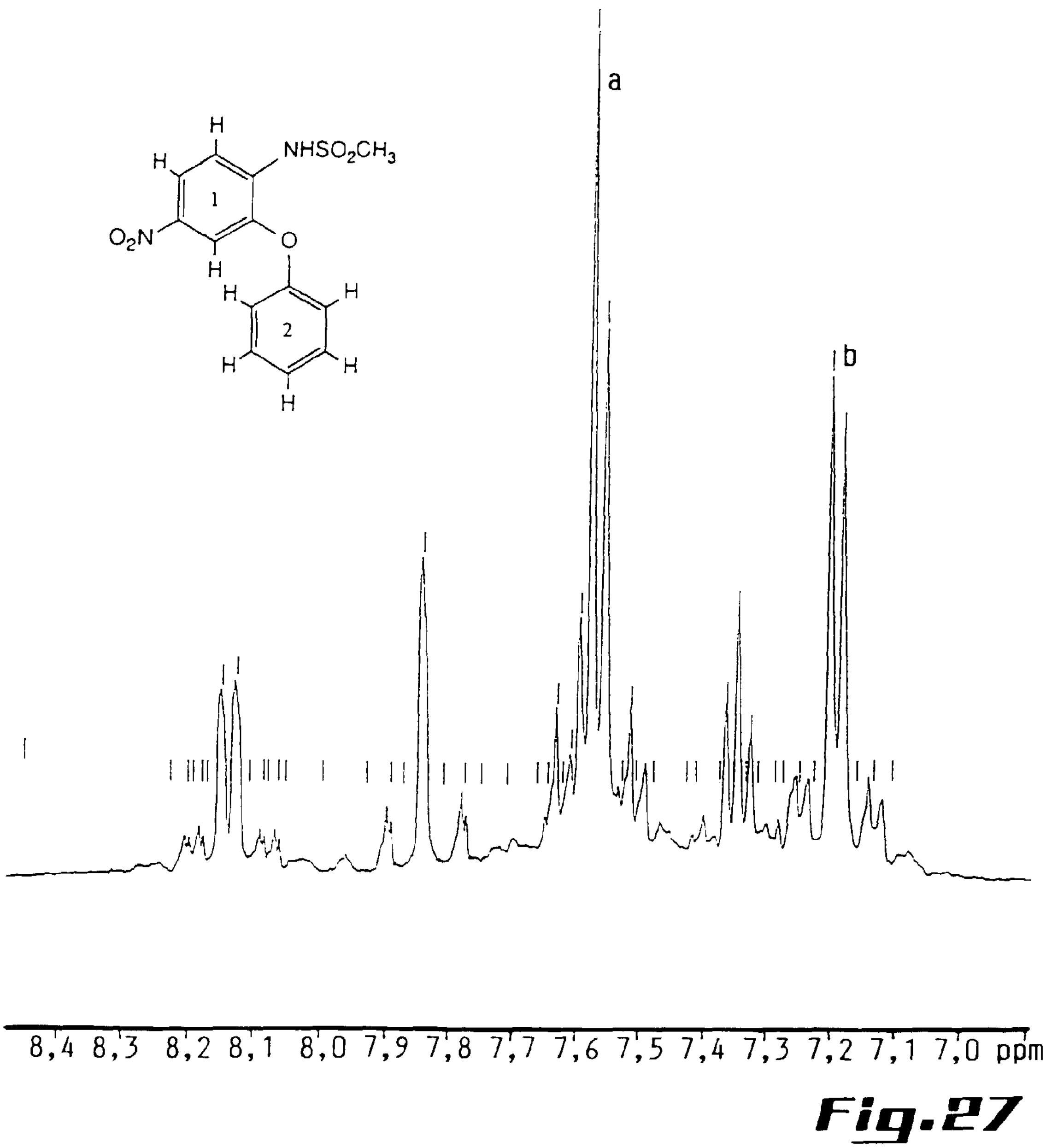


Fig.27

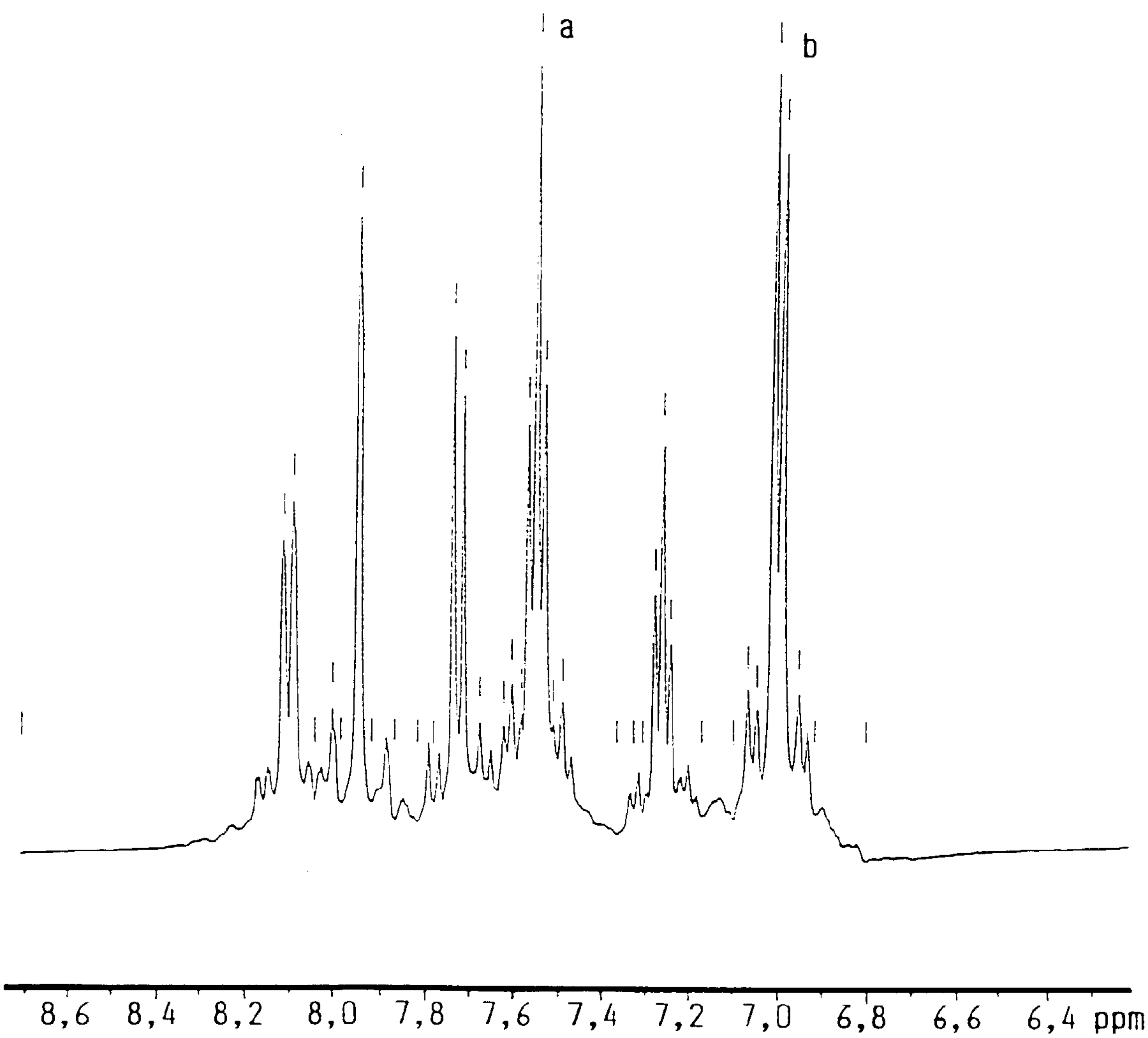


Fig.28